(19) Europäisches Patentamt
European Patent Office
Office européen des brevets

(11) **EP 2 746 398 A1**

(12) **EUROPEAN PATENT APPLICATION**


(43) Date of publication:
**25.06.2014 Bulletin 2014/26**

(21) Application number: **12199104.6**

(22) Date of filing: **21.12.2012**

(51) Int Cl.:
***C12P 7/64*** *(2006.01)* ***C12R 1/01*** *(2006.01)*
***C12R 1/225*** *(2006.01)* ***A23L 1/30*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**



(71) Applicant: **LABORATORIOS ORDESA, S.L**
**08830 Sant Boi de Llobregat, Barcelona (ES)**

(72) Inventors:
- **Moreno Muñoz, José Antonio**
  **08222 TERRASSA (ES)**
- **Rivero Urgell, Montserrat**
  **08035 BARCELONA (ES)**
- **Cifuentes Orjuela, Gloria Clemencia**
  **08025 BARCELONA (ES)**
- **Martín Garciá, Francisco José**
  **08041 BARCELONA (ES)**
- **Rodríguez-Palmero Seuma, María**
  **08029 BARCELONA (ES)**
- **Puigjaner Riba, Joaquim**
  **08794 LES CABANYES (ES)**
- **Villar Tajadura, María Antonia**
  **28049 MADRID (ES)**
- **Rodríguez Alcalá, Luis Miguel**
  **28049 MADRID (ES)**
- **Requena Rolania, Teresa**
  **28049 MADRID (ES)**
- **Fontecha Alonso, Francisco Javier**
  **28049 MADRID (ES)**

(74) Representative: **ZBM Patents - Zea, Barlocci & Markvardsen**
**Zea, Barlocci & Markvardsen**
**Plaza Catalunya, 1**
**08002 Barcelona (ES)**


(54) **Process for producing conjugated linolenic acid from linolenic acid employing Bifidobacterium breve, Bifidobacterium bifidum, or Lactobacillus oris strains.**


(57) Method for preparing isomers of conjugated linolenic acid (CLNA) from linolenic acid (LNA) in a medium comprising at least the fat portion contained in a milk-based product or composition, in which at least one strain of a lactic acid bacteria or a bifidobacteria selected from the species of the group consisting of *Bifidobacterium breve, Bifidobacterium bifidum* and *Lactobacillus oris* is used. The invention also includes particular strains of all these species that allow performing the method in with high yields and conversion rates, as well as food products, nutritional compositions and pharmaceutical compositions containing them which are useful as anti-obesity agents and as immunomodulator agents.


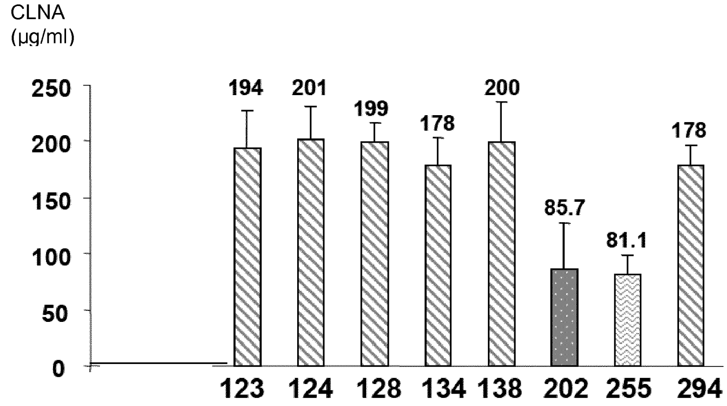


**FIG. 3**

EP 2 746 398 A1

## Description

The present invention relates to a method for obtaining or preparing isomers of the conjugated linolenic acid (CLNA), and to lactic acid bacteria and bifidobacteria for carrying out said method.

## BACKGROUND ART

Conjugated linolenic acid (CLNA) is a mixture of geometrically and positional isomers of α-linolenic acid (C18:3, *cis*-9 *cis*-12 *cis*-15, LNA,) with conjugated double bonds. There are a lot of isomers, some of them being present in seed oil, such as C18:3 *cis*-9, *trans*-11, *cis*-13, and other being generated by biohydrogenation with ruminal bacteria, which can be found in milk and meat (as rumelenic acid C18:3 *cis* 9, *trans* 11, *cis* 15 and isorumelenic acid C18:3 *cis* 9, *trans* 13, *cis* 15).

With regard to the biological properties of the CLNA isomers, it has been reported the use as anti-inflammatory agent, anti-obesity, antitumor agent, anti-aterogenic and as immunomodulatory agent.

In the last years it has been disclosed that some isomers of CLNA are produced due to the metabolism of lactic acid bacteria. Thus, isomers like C18:3 *cis*-9 *trans*-11 *cis*-15 CLNA, and C18:3 *trans*-9 *trans*-11 *cis*-15 CLNA have been produced *in vitro* by bacteria in bacterial culture media. For example, the document Gorissen et al, "Production of conjugated linoleic and linolenic acid isomers by Bifidobacterium species", Appl. Microbiol Biotechnol - 2010, vol. 87, pp.: 2257-2266, discloses some strains of *Bifidobacterium* able to transform in Man, Rogosa and Sharpe (MRS) broth linoleic acid (LA) and linolenic acid (LNA) into conjugated linoleic (CLA) and linolenic acids (CLNA), respectively, with conversion rates from 19 % to 54 % for CLA, and comprised from 55 % to 78 % for CLNA.

Bacterial production of CLNA from LNA is critical because LNA is considered a toxic for some bacterial cells, in such a way that the growth of the cell culture is retarded. Thus, it is difficult to obtain great amounts of CLNA and with high yields from bacteria. Indeed, bacterial transformation of LNA to CLNA is a defense mechanism against toxic agents.

Nonetheless, there are bacterial strains that provide high conversion rates in MRS medium when LNA is added into said medium. For example, the strain *Bifidobacterium breve* DPC6330 disclosed in Hennessy et al., "The Production of Conjugated α-Linolenic, γ-Linolenic and Stearidonic Acids by Strains of Bifidobacteria and Propionibacteria", Lipids - 2012, vol. 47, pp: 313-327. The strain of *Bifidobacterium breve* DPC6330 was able to convert in MRS the 90 % of the added LNA.

These bacterial producers of CLNA, most of them being commensally bifidobacteria or from dairy products, are of great interest because they represent possible sources for the delivery of conjugated fatty acids in mammals (in particular human) gastrointestinal tract (GIT).

The provision of compositions containing CLNA to organisms, by means of functional foods, dairy products (milk derived products), nutritional compositions or pharmaceutical compositions, represents an interesting challenge giving raise to efficient added-value products.

Nonetheless, it is widely accepted in the art that conversion rates in a particular medium cannot be extrapolated to other media, in particular to fermented foods and dairy products. So then, in the Short Communication document Gorissen et al., "Microbial production of conjugated linoleic and linolenic acids in fermented foods: Technological bottlenecks", Eur. J. Lipid Sci. Technol. - 2012, vol. 114, pp. 486-491, it is concluded that application of bacterial cultures for enhanced concentrations of CLA and CLNA in fermented foods is not straightforward. In this document some bifidobacteria and a *Lactobacillus sakei* strain, all of them able to produce CLA and CLNA *in vitro* (MRS), were applied as starter cultures for the fermentation of milk and meat, respectively. However no increase in the CLA and CLNA content was obtained.

Therefore, there is a need of alternative methods for the production of these conjugated fatty acids, which methods promote high yields, or conversion rates. In addition, it is desirable the provision of compositions with high amounts of CLNA, as well as of compositions comprising the precursor ingredients for finally obtaining *in situ* effective amounts of CLNA.

## SUMMARY OF THE INVENTION

Inventors have found that some strains used in dairy or agroalimentary industry, in particular strains of Lactic Acid Bacteria (LAB) and Bifidobacteria, selected from the species of the group consisting of *Bifidobacterium breve, Bifidobacterium bifidum,* and *Lactobacillus oris,* are able to produce a high percentage of conversion of linolenic acid (LNA) to conjugated linolenic acid (CLNA) in several media, including edible compositions with milk-based products from animal or vegetal origin, ingredients or compounds, the media containing or being supplemented with linolenic acid, which is generally a toxic for the bacterial cells.

According to the inventor's knowledge, this is the first time that there have been isolated lactic acid bacteria and bifidobacteria that can grow and produce CLNA in several media with high purity and yields.

The inventors provide also a method for the production of CLNA from LNA with high conversion rates, in which the specific lactic acid bacteria and bifidobacteria are used to promote such conversion. As above indicated, the isolated strains of lactic acid bacteria and bifidobacteria are able to promote the conversion in different media, including food matrices, such as food products, or nutritional formulas, in particular in milk-based nutritional formulas, as well as in other type of milk-based compositions. This is the first time that bacterial production of CLNA from LNA in a medium representing an edible product has been achieved, due to the fact that it has been surprisingly found specific lactic acid bacteria and bifidobacteria strains that, once in contact with LNA in a medium comprising a milk-based product, are able to produce isomers of the conjugated linolenic acid with high yields or conversion rates. In addition, the method allows obtaining with a high purity one of the isomers of CLNA, in particular the isomer C18:3 *cis*-9 *trans*-11 *cis*-15 conjugated linolenic acid. With this method it has been achieved that a medium, which can be an edible composition comprising at least the fat portion (from animal or vegetal origin) contained in a milk-based product, said milk-based product from animal or vegetal origin, such as a yogurt, a fermented milk, an infant formula or food supplement could be enriched with CLNA and provides thus the beneficial effects associated to this compound.

Thus, in a first aspect the invention aims a method for preparing an isomer of conjugated linolenic acid (CLNA) from linolenic acid (LNA) in a medium comprising at least the fat portion, from animal or vegetal origin, contained in a milk-based product, said fat portion containing linolenic acid, the method comprising adding to the medium at least one strain used in dairy or agroalimentary industry, selected from the group consisting of a lactic acid bacteria (LAB) and bifidobacteria, said strains characterized by:

i) being able to grow to an optical density of at least 0.9 $OD_{600}$ determined at 48 hours after initial of growing in anaerobiosis, in a Man Rogosa Sharpe broth medium, as well as in a medium comprising at least the fat portion, from animal or vegetal origin, contained in a milk-based product, both media comprising at least 0.3 mg/ml of linolenic acid in the medium; and

ii) being able to produce conjugated linolenic acid (CLNA) in both media.

Advantageously, the method provides a conversion rate in percentage by weight of linolenic acid to conjugated linolenic acid comprised from 30 % (w/w) to 100 % (w/w), said conversion ratio calculated with the following formula:

$$\% \text{ of CLNA} = \text{weight of CLNA} / (\text{weight of CLNA} + \text{weight of LNA}) \times 100.$$

Another aspect of the invention is a bifidobacteria strain, which is a strain of *Bifidobacterium breve* deposited by the Depositor in the Colección Española de Cultivos Tipo (CECT) under the Depositor's reference ORD0123, or a mutant or variant thereof.

The strain of *Bifidobacterium breve* of the invention, isolated from infant (0-3 months) faeces from Spain was deposited, according to the Budapest Treaty, on the 29th November 2012 in the Colección Española de Cultivos Tipo (CECT) in the Universidad de Valencia C.P 46980 Catedrático Agustín Escardino N° 9 Paterna, Valencia (Spain) (former in the Universidad de Valencia C.P 46100 Burjasot, Valencia (Spain)), by the depositor Laboratorios ORDESA, S.L. (C/ Osca, 18-20, 08830 Sant Boi de Llobregat-Spain). The strain of *Bifidobacterium breve* was identified by the depositor with the reference ORD0123, and received the provisional CECT accession number CECT 8241.

Another aspect of the invention is a bifidobacteria strain, which is a strain of *Bifidobacterium breve* deposited in the Colección Española de Cultivos Tipo (CECT) under the Depositor's reference ORD0124, or a mutant or variant thereof.

The strain of *Bifidobacterium breve* of the invention, isolated from infant (0-3 months) faeces from Spain was deposited, according to the Budapest Treaty, on the 29th November 2012 in the Colección Española de Cultivos Tipo (CECT) in the Universidad de Valencia C.P 46980 Catedrático Agustín Escardino N° 9 Paterna, Valencia (Spain) (former in the Universidad de Valencia C.P 46100 Burjasot, Valencia (Spain)), by the depositor Laboratorios ORDESA, S.L. (C/ Osca, 18-20, 08830 Sant Boi de Llobregat-Spain). The strain of *Bifidobacterium breve* was identified by the depositor with the reference ORD0124, and received the provisional CECT accession number CECT 8242.

Another aspect of the invention is a bifidobacteria strain, which is a strain of *Bifidobacterium breve* deposited in the Colección Española de Cultivos Tipo (CECT) under the Depositor's reference ORD0128, or a mutant or variant thereof.

The strain of *Bifidobacterium breve* of the invention, isolated from infant (0-3 months) faeces from Spain was deposited, according to the Budapest Treaty, on the 29th November 2012 in the Colección Española de Cultivos Tipo (CECT) in the Universidad de Valencia C.P 46980 Catedrático Agustín Escardino N° 9 Paterna, Valencia (Spain) (former in the Universidad de Valencia C.P 46100 Burjasot, Valencia (Spain)), by the depositor Laboratorios ORDESA, S.L. (C/

Osca, 18-20, 08830 Sant Boi de Llobregat-Spain). The strain of *Bifidobacterium breve* was identified by the depositor with the reference ORD0128, and received the provisional CECT accession number CECT 8239.

The invention also aims a bifidobacteria strain, which is a strain of *Bifidobacterium breve* deposited in the Colección Española de Cultivos Tipo (CECT) under the Depositor's reference ORD0134, or a mutant or variant thereof.

The strain of *Bifidobacterium breve* of the invention, isolated from infant (0-3 months) faeces from Spain was deposited, according to the Budapest Treaty, on the 29th November 2012 in the Colección Española de Cultivos Tipo (CECT) in the Universidad de Valencia C.P 46980 Catedrático Agustín Escardino N° 9 Paterna, Valencia (Spain) (former in the Universidad de Valencia C.P 46100 Burjasot, Valencia (Spain)), by the depositor Laboratorios ORDESA, S.L. (C/ Osca, 18-20, 08830 Sant Boi de Llobregat-Spain). The strain of *Bifidobacterium breve* was identified by the depositor with the reference ORD0134, and received the provisional CECT accession number CECT 8243.

Another aspect of the invention is a bifidobacteria strain, which is a strain of *Bifidobacterium breve* deposited in the Colección Española de Cultivos Tipo (CECT) under the Depositor's reference ORD0138, or a mutant or variant thereof.

The strain of *Bifidobacterium breve* of the invention, isolated from infant (0-3 months) faeces from Spain was deposited, according to the Budapest Treaty, on the 29th November 2012 in the Colección Española de Cultivos Tipo (CECT) in the Universidad de Valencia C.P 46980 Catedrático Agustín Escardino N° 9 Paterna, Valencia (Spain) (former in the Universidad de Valencia C.P 46100 Burjasot, Valencia (Spain)), by the depositor Laboratorios ORDESA, S.L. (C/ Osca, 18-20, 08830 Sant Boi de Llobregat-Spain). The strain of *Bifidobacterium breve* was identified by the depositor with the reference ORD0138, and received the provisional CECT accession number CECT 8244.

Another aspect of the invention is a bifidobacteria strain, which is a strain of *Bifidobacterium breve* deposited in the Colección Española de Cultivos Tipo (CECT) under the Depositor's reference ORD0294, or a mutant or variant thereof.

The strain of *Bifidobacterium breve* of the invention, isolated from infant (0-3 months) faeces from Spain was deposited, according to the Budapest Treaty, on the 29th November 2012 in the Colección Española de Cultivos Tipo (CECT) in the Universidad de Valencia C.P 46980 Catedrático Agustín Escardino N° 9 Paterna, Valencia (Spain) (former in the Universidad de Valencia C.P 46100 Burjasot, Valencia (Spain)), by the depositor Laboratorios ORDESA, S.L. (C/ Osca, 18-20, 08830 Sant Boi de Llobregat-Spain). The strain of *Bifidobacterium breve* was identified by the depositor with the reference ORD0294, and received the provisional CECT accession number CECT 8246.

Another aspect of the invention is a bifidobacteria strain, which is a strain of *Bifidobacterium bifidum* deposited in the Colección Española de Cultivos Tipo (CECT) under the Depositor's reference ORD0202, or a mutant or variant thereof.

The strain of *Bifidobacterium bifidum* of the invention, isolated from infant (0-3 months) faeces from Spain was deposited, according to the Budapest Treaty, on the 29th November 2012 in the Colección Española de Cultivos Tipo (CECT) in the Universidad de Valencia C.P 46980 Catedrático Agustín Escardino N° 9 Paterna, Valencia (Spain) (former in the Universidad de Valencia C.P 46100 Burjasot, Valencia (Spain)), by the depositor Laboratorios ORDESA, S.L. (C/ Osca, 18-20, 08830 Sant Boi de Llobregat-Spain). The strain of *Bifidobacterium bifidum* was identified by the depositor with the reference ORD0202, and received the provisional CECT accession number CECT 8245.

Yet another aspect of the invention is a Lactic acid bacteria strain, which is a strain of *Lactobacillus oris* deposited in the Colección Española de Cultivos Tipo (CECT) under the Depositor's reference ORD0255, or a mutant or variant thereof.

The strain of *Lactobacillus oris* of the invention, isolated from breastmilk (*from a Spanish woman*) was deposited, according to the Budapest Treaty, on the 29th November 2012 in the Colección Española de Cultivos Tipo (CECT) in the Universidad de Valencia C.P 46980 Catedrático Agustín Escardino N° 9 Paterna, Valencia (Spain) (former in the Universidad de Valencia C.P 46100 Burjasot, Valencia (Spain)), by the depositor Laboratorios ORDESA, S.L. (C/Osca, 18-20, 08830 Sant Boi de Llobregat- Spain). The strain of *Lactobacillus oris* was identified by the depositor with the reference ORD0255, and received the provisional CECT accession number CECT 8240.

*Bifidobacterium bifidum* ORD0202 (provisional CECT 8245), *Bifidobacterium breve* ORD0123 (provisional CECT 8241), *Bifidobacterium breve* ORD0124 (provisional CECT 8242), *Bifidobacterium breve* ORD0128 (provisional CECT 8239), *Bifidobacterium breve* ORD0134 (provisional CECT 8243), *Bifidobacterium breve* ORD0138 (provisional CECT 8244), *Bifidobacterium breve* ORD0294 (provisional CECT 8246) were isolated from baby faeces (from Spain origin) and *Lactobacillus oris* ORD0255 (provisional CECT 8240) from human breast milk (from Spain origin). The taxonomic characterization was performed as indicated in the Examples, namely by sequencing almost full sequence of the 16S rRNA.

The invention also includes mutants or variants of these strains, wherein the mutants or variants of said strains are obtained using one of the deposited strains as starting material, and wherein the mutant strains retain the essential properties of the deposited strains, wherein said essential properties are:

i) being able to grow to an optical density of at least 0.9 $OD_{600}$ determined at 48 hours after initial of growing in anaerobiosis, in a Man Rogosa Sharpe broth medium, as well as in a medium comprising at least the fat portion, from animal or vegetal origin, contained in a milk-based product, both media comprising at least 0.3 mg/ml of linolenic acid in the medium, and

ii) being able to produce conjugated linolenic acid (CLNA) in both media.

Another aspect of the invention is a bacterial pure culture obtained from any of the strains as defined above. These pure cultures are obtained using the standard microbiological processes.
Yet another aspect of the invention is a food product which comprises an effective amount (also named nutritionally effective amount) of at least one of the strains of the invention, together with appropriate amounts of other edible ingredients, and LNA.
In another aspect, the invention relates to a nutritional composition which comprises a nutritionally effective amount of at least one of the strains of the invention as defined above, and LNA, together with appropriate amounts of other appropriate edible ingredients.
In another aspect, the invention relates to a pharmaceutical composition which comprises a therapeutically effective amount of at least one of the strains as defined above, and LNA, together with appropriate amounts of pharmaceutical acceptable excipients and/or carriers.
Another aspect of the invention relates to any of the strains as defined above, for use as a probiotic.
A further aspect of the invention relates to any of the strains as defined above, for use as anti-obesity agent.
This aspect can be formulated as the use of any of the strains of invention, for the manufacture of a medicament, a food product or a nutritional composition for the treatment and/or prevention of obesity in an animal including a human. The present invention also relates to a method for the treatment or prevention of obesity, comprising administering a therapeutically effective amount of any of the strains as defined above, or a pharmaceutical composition, or a food product or a nutritional composition comprising at least any of the strains, together with pharmaceutically acceptable excipients or carriers, or edible ingredients in a subject in need thereof, including a human.
Another aspect of the invention is the use of the LAB and Bifidobacteria strains of the invention for the production of vitamins in a medium comprising at least the fat portion (from animal or vegetal origin) contained in a milk-based product, said fat portion comprising linolenic acid.
In a final aspect, the invention relates to any of the strains as defined above, for use as immunomodulator agent.
This final aspect can be formulated as the use of any of the strains of invention, for the manufacture of a medicament, a food product or a nutritional composition for the therapeutic and/or prevention of immune system diseases of an animal including a human. The invention may alternatively be formulated as a method for the treatment or prevention of immune system diseases, comprising administering a therapeutically effective amount of any of the strains as defined above, or a pharmaceutical composition, or a food product or a nutritional composition comprising at least one of the strains, together with pharmaceutically acceptable excipients or carriers, or edible ingredients in a subject in need thereof, including a human. All these compositions aim the modulation of the immune system.

BRIEF DESCRIPTION OF THE DRAWINGS

FIG. 1, related to Example 1, is a bar diagram showing in the Y-axis the percentage of conversion (conversion rate, %) of LNA to CLNA in a medium comprising a reconstituted skimmed milk as milk-based product. The number under the bars (X-axis) relate to the inoculated strains *Lactobacillus oris* ORD0255 (identified as 255), *Bifidobacterium breve* ORD0123 (identified as 123), *Bifidobacterium breve* ORD0124 (identified as 124), *Bifidobacterium breve* ORD0128 (identified as 128), *Bifidobacterium breve* ORD0134 (identified as 134), *Bifidobacterium breve* ORD0138 (identified 138), *Bifidobacterium breve* ORD0294 (identified as 294), *Bifidobacterium bifidum* ORD0202 (identified as 202).

FIG. 2, related to Example 1, is another bar diagram, in which it is indicated the percentage by weight (% in the Y-axis) of the isomer C18:3 *cis*-9 *trans*-11 cis-15 of the conjugated linolenic acid obtained in FIG. 1. X-axis indicates the Depositors's reference of the strains as in FIG. 1.

FIG. 3, related to Example 1, shows in the Y-axis the concentration in micrograms/ml of CLNA obtained using as a bacterial medium an infant formula (Blemil-Plus-1 Forte@, LABORATORIOS ORDESA, S.L.). The number under the bars relate to the inoculated strains *Lactobacillus oris* ORD0255 (identified as 255), *Bifidobacterium breve* ORD0123 (identified as 123), *Bifidobacterium breve* ORD0124 (identified as 124), *Bifidobacterium breve* ORD0128 (identified as 128), *Bifidobacterium breve* ORD134 (identified as 134), *Bifidobacterium breve* ORD0138 (identified as 138), *Bifidobacterium breve* ORD0294 (identified as 294), *Bifidobacterium bifidum* ORD0202 (identified as 202).

DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition. The following definitions are included for the purpose of understanding

For "strain used in dairy or agroalimentary industry" is to be understood any strain of a bacteria that is traditionally known as a "food-grade bacteria", which means that can be used in the processes for obtaining food, or as active principles in any edible composition. Among strains used in dairy (milk) or agroalimentary industry there are the Bifidobacteria and the Lactic Acid Bacteria (LAB), including among others the genus Lactobacillus, and Streptococcus.

For "milk or dairy derivative" or "milk-based compound" or "milk-based product", used herewith interchangeable, is to be understood any composition or compound derived from milk of any source and origin, including animal or vegetal milk, such as milk (entire, skimmed, semi-skimmed) in liquid form or as a powder, a yogurt, a curd, a cheese, an infant formula, and an ice-cream. Said dairy product can be used as an ingredient of a composition containing other edible ingredients, as well as a source of carbon or other compounds, namely as a source of linolenic acid. The "fat portion" or "fat fraction" (interchangeable herewith) contained in a milk or a milk-based product is to be understood as the fraction of lipidic components (from animal or vegetalorigin) in any milk type or product containing milk. Initially milk fat is secreted in the form of a fat globule surrounded by a membrane. Each fat globule is composed almost entirely of triacylglycerols and is surrounded by a membrane consisting of complex lipids such as phospholipids, along with proteins. These act as emulsifiers which keep the individual globules from coalescing and protect the contents of these globules from various enzymes in the fluid portion of the milk. Although 97-98% of lipids are triacylglycerols, small amounts of di- and monoacylglycerols, free cholesterol and cholesterol esters, free fatty acids, and phospholipids are also present. Unlike protein and carbohydrates, fat composition in milk varies widely in the composition due to genetic, lactation, and nutritional factor difference between different species. The fat-soluble vitamins A, D, E, and K along with essential fatty acids such as linoleic acid and linolenic acid are also found within the milk fat portion, from animal or vegetal origin, of the milk. Alternatively the origin of a "fat portion" or "fat fraction" (interchangeable herewith) contained in a milk or a milk-based product is to be understood as the fraction of lipidic components that could be from vegetal origin, which is for example, added in some infant formulas.

By the expression "enzymatic activity profile" is to be understood the pool of enzymes that are active in a cell system, in particular in a bacterial strain, and which give raise to a determined phenotype.

The term "probiotic" is to be understood in the sense of the present invention as live microorganisms which when administered in adequate amounts confer a health benefit on the host. The known benefits of enteral administration of probiotic microorganisms include enhanced host defense to disease improving the properties of the indigenous microbiota and increasing colonization resistance to the harmful microbiota. Probiotics have been suggested to play an important role in the formation or establishment of a well-balanced, indigenous, intestinal microbiota in newborn children or adults receiving high doses of antibiotics. Lactic acid bacteria, especially specific strains of *Lactobacillus* and species of *Streptococcus, Enterococcus* and *Bifidobacterium* genera have been recommended for their use as probiotics.

A "mutant of a bacterial strain or variant thereof" encompasses bacteria obtained by mutation, variation or recombination of each of the strains, provided that the resulting bacteria have the activity of growing to an optical density of at least 0.9 (said optical density determined at 600 nm, $OD_{600}$ , and at 48 hours after initial of growing in anaerobiosis), in a Man Rogosa Sharpe broth medium, as well as in a medium comprising at least the fat portion, from animal or vegetal origin, contained in a milk-based product, both media comprising at least 0.3 mg/ml of linolenic acid in the medium; and being able to produce conjugated linolenic acid (CLNA) in both media. In particular embodiments of the invention, the mutant is a genetically modified mutant obtained by classical mutagenesis (i.e. using chemical or physical agents) or by genetic engineering techniques. In another embodiment, the variant is a naturally occurring variant.

The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc., must also be "acceptable" in the sense of being compatible with the other ingredients of the formulation. Suitable carriers, excipients, etc. can be found in standard pharmaceutical texts, and include, as a way of example preservatives, agglutinants, humectants, emollients, and antioxidants.

The term "therapeutically effective amount" or "effective amount" as used herein, means an amount of an active agent (ingredient) high enough to deliver the desired benefit, either the treatment or prevention of the illness, but low enough to avoid serious side effects within the scope of medical judgment.

For "anti-obesity agent" is to be understood any compound or product (including pharmaceutical active principles, and bacterial strains *per se* or as ingredients in pharmaceutical compositions), which prevents obesity or even allows treating obesity.

The term "immunomodulator agent" relates to any compound, bacterial strain or composition that is able to promote or to regulate the balanced functioning of the immune system. For balanced functioning is to be understood that the system does not lead to autoimmune diseases, as well as does not conduct to any immune-depressed situation.

For the purposes of the invention, any ranges given include both the lower and the upper end-points of the range.

As will be illustrated below, the method of the invention allows obtaining high amounts of the isomers of CLNA.

In an embodiment of the invention, the bacillus strains of lactic acid bacteria and of bifidobacteria used in the method are further characterized by comprising the following enzymatic activity profile:

Leucine arilamidase (+)
β-galactosidase (+)
Lipase (-)
Trypsin (-)
Chymotrypsin (-)
β-glucuronidase (-),

wherein (+) means that the enzymatic activity is present, and (-) means that the enzymatic activity is not present; said enzymatic activity measured performing the following steps:

i) filling a well in a microtiter plate with enzymatic substrates and buffers;
ii) adding the bacterial suspensions;
iii) incubating the mixtures at 37 °C under atmospheric pressure;
iv) adding a colour reagent containing 20 mg Fast Blue BB; and
v) keeping in dark for 5 minutes and exposing the mixture to a 750-watt lamp to prevent non-specific yellowing of the colour reagent.

The determination of this enzymatic activity is usually automatically performed using the commercial system, API ZYM (API system, BioMerieux), and following the manufacturer's instructions.

The determination of the growing of the LAB and Bifidobacteria strains is performed by determining the Optical Density (OD) with a spectrophotometer at a wavelength of 600 nm of a growing culture comprising the desired medium (MRS or a medium with a milk-based product) and the strain (inoculums at 2 % v/v from a bacterial culture grown 48 hours in the same media) and incubating the mixture at 37 °C in anaerobiosis. The determination of the optical density of the growing culture is done at 48 hours after initial of the culture growing.

In an embodiment of the method of the invention, the linolenic acid comprised in the media (MRS or medium comprising at least the fat portion, from animal or vegetal origin, contained in a milk-based product) is provided as a supplement of the media or it is a constituent of the elected media, in particular it can be used the linolenic acid provided in the at least the fat portion (from animal or vegetal origin) contained as component or ingredient in a milk-based product.

In another embodiment, the method is performed in a medium comprising a milk-based product selected from the group consisting of milk (entire, skimmed, semi-skimmed) in liquid form or as a powder, a yogurt, a curd, a cheese, an ice-cream, a milk infant formula, and mixtures thereof. In a most preferred embodiment the milk-based product is a reconstituted powder milk, in particular a reconstituted skim milk powder. In another preferred embodiment, the milk-based product is a milk infant formula. The milk-based product can be of any origin, such as milk from cow, goat, or sheep. Indeed, the milk-based product has to comprise at least a fat portion (from animal or vegetal origin).

In another preferred embodiment, when the milk-based product is a reconstituted powder milk, in particular a reconstituted skimmed milk, the conversion rate is comprised from 60 % to 99 % using any of the bacteria selected from LAB or bifidobacteria strains of the species *Bifidobacterium breve, Bifidobacterium bifidum* and *Lactobacillus oris.*

When the method of the invention is performed in a milk infant formula, the conversion rate is comprised from 30 % to 80 %. In an embodiment, when the strain is of *Bifidobacterium breve,* the conversion rate of LNA to CLNA in a milk infant formula is comprised from 70 % to 80 %.

In another embodiment of the method of the invention, the conjugated linolenic acid obtained is the isomer C18:3 *cis*-9 *trans*-11 *cis*-15. This isomer, in a mixture with the isomer *cis*-9 *trans*-13 *cis*-15 has been reported as anti-obesity agent due to its capacity of increasing lipid mobilization in adipose tissue.

In a preferred embodiment, the percentage by weight of the isomer C18:3 *cis*-9 *trans*-11 *cis*-15 conjugated linolenic acid is comprised from 75 % (w/w) to 98 % (w/w) referred to the total amount of conjugated linolenic acid obtained in the method.

In an embodiment of the method of the invention the mass or amount (weight) of CLNA isomers were measured by gas chromatography after direct transmethylation procedure of the sample to obtain the fatty acid methyl esters and using C17:0 as internal standard.

In an embodiment of the method of the invention, the strain used in dairy or agroalimentary industry is a strain of *Bifidobacterium* with the ability to grow in both media: MRS, and a media comprising at least the fat portion (from animal or vegetal origin) or fraction contained as a component of the milk-based product, both media also comprising free LNA, which is added as a supplement or is contained in the at least the fat portion (from animal or vegetal origin) of the milk-based product. The condition is that the LAB or Bifidobacteria is able to grow in a medium with a concentration of LNA of at least 0.3 mg/ml (w/v) in respect of the total volume wherein the analysis of the growing of the bacteria is performed. A preferred amount is selected from the group consisting of 0.3 mg/ml, 0.4 mg/ml, 0.5 mg/ml, 0.6 mg/ml, 0.7 mg/ml and 0.8 mg/ml. In a most preferred embodiment the concentration of LNA is 0.5 mg/ml. In a particular embodiment the strain of Bifidobacterium is selected from *Bifidobacterium breve* and *Bifidobacterium bifidum.*

In another embodiment of the invention the strain used in dairy or agroalimentary industry is a LAB strain, in particular is a strain of *Lactobacillus* with the ability to grow in both media MRS and a media comprising a milk-based product, both media also comprising LNA in a concentration of at least 0.3 mg/ml (w/v) in respect of the total volume wherein the analysis of the growth of the bacteria is performed. A preferred amount is selected from the group consisting of 0.3 mg/ml, 0.4 mg/ml, 0.5 mg/ml, 0.6 mg/ml, 0.7 mg/ml and 0.8 mg/ml. In a particular embodiment the strain is of *Lactobacillus oris.*

Another embodiment of the invention is a method as disclosed above, wherein the strain is selected from the group consisting of:

- *Bifidobacterium breve* deposited in the Colección Española de Cultivos Tipo (CECT) under the Depositor's reference ORD0123, provisional identified as CECT 8241, or a mutant or variant thereof;
- *Bifidobacterium breve* deposited in the Colección Española de Cultivos Tipo (CECT) under the Depositor's reference ORD0124, provisional identified as CECT 8242 or a mutant or variant thereof;
- *Bifidobacterium breve* deposited in the Colección Española de Cultivos Tipo (CECT) under the Depositor's reference ORD0128, provisional identified as CECT 8239 or a mutant or variant thereof;
- *Bifidobacterium breve* deposited in the Colección Española de Cultivos Tipo (CECT) under the Depositor's reference ORD0134, provisional identified as CECT 8243, or a mutant or variant thereof;
- *Bifidobacterium breve* deposited in the Colección Española de Cultivos Tipo (CECT) under the Depositor's reference ORD0138, provisional identified as CECT 8244, or a mutant or variant thereof;
- *Bifidobacterium breve* deposited in the Colección Española de Cultivos Tipo (CECT) under the Depositor's reference ORD0294, provisional identified as CECT 8246 or a mutant or variant thereof;
- *Bifidobacterium bifidum* deposited in the Colección Española de Cultivos Tipo (CECT) under the Depositor's reference ORD202, provisional identified as CECT 8245, or a mutant or variant thereof; and
- *Lactobacillus oris* deposited in the Colección Española de Cultivos Tipo (CECT) under the Depositor's reference ORD255, provisional identified as CECT 8240, or a mutant or variant thereof,

wherein the mutants or variants of said strains are obtained using one of the deposited strains as starting material, and wherein the mutant strains retain the essential properties of the deposited strains, wherein said essential properties are:

i) the ability to grow to an optical density of at least 0.9 ($OD_{600}$), determined at 48 hours after initial of growth in anaerobiosis, in a Man Rogosa Sharpe broth medium, as well as in a medium comprising at least the fat portion, from animal or vegetal origin, contained in a milk-based product, both media comprising at least 0.3 mg/ml of linolenic acid in the medium; and

ii) being able to produce conjugated linolenic acid (CLNA) in both media.

All the above-mentioned bacterial strains are strains used in dairy or agroalimentary industry, including the lactic acid bacteria (LAB), such as Lactobacillus, and Bifidobacteria. All the strains used in the method have the advantage of being able to produce CLNA in high amounts in several media, including not only *in vitro* growth bacterial media, but also in products derived from milk or milk-based products. This implies the additional advantage of being usable in compositions or in edible products containing milk that are further consumed by animals, including human. In addition all the strains used in the method of the invention, in particular those strains of Bifidobacteria and Lactobacillus have a bacillus morphology or shape, that is, they are rod-shape bacteria.

All these bacteria can be used in the method of the invention, in which the source of free LNA is from the milk-based product itself (fat portion or fraction from animal or vegetal origin), or can be externally provided from other source or environment. Examples include linseed (flaxseed), soybean, rapeseed (canola), and walnuts.

Independently of the source of LNA, the strains can produce CLNA in several media, for example in the context of a milk composition in the gastrointestinal tract.

In addition, the strains of the invention are producers of highly pure isomers of CLNA, in particular of an isomer that has been associated to the reduction of obesity risk. See for reference Miranda et al., "cis-9, trans-11, cis-15 and cis-9, trans-13, cis-15 CLNA mixtures activates PPARα in HEK293 and reduces triacylglycerols in 3T3-L1 cells", Lipids -2011, vol. 46(11), pp.: 1006-1012.

Furthermore, the strains according to the invention behave the additional advantage that they are also producers of other conjugated fatty acid isomers, such as C18:2 *cis*-9, *trans*-11 conjugated linoleic acid (Rumenic acid, CLA) and low amounts of C18:2 *trans*-10, *cis*-12, with interesting properties. Among said interesting properties, CLA has been mainly related with increased immune function, but also as an anti-cancer agent or agent helpful against cancer (colorectal cancer), and as anti-atherosclerosis agent useful in cardiovascular diseases, for treating high blood pressure, high cholesterol and triacylglycerides levels, osteoporosis, insulin resistance, inflammation, food-induced allergic reactions and as agent for modulating body composition (lowering body fat, preserving muscle tissue).

Besides, as will be illustrated in the examples below, the strains produce other interesting compounds for health like vitamins. Some of the vitamins produced include vitamin B9 and B12. Vitamin B9 is reported as immune modulator agent or immune enhancing agent, meanwhile vitamin B12, among others properties, is also employed as anti-obesity agent. Vitamin B9 deficiency during pregnancy can produce pregnancy complications including neural tube defects, increased risk of congenital heart defects, increased risk of preterm delivery, infant low birth weight and fetal growth retardation, also B9 deficiency has been related with increasing homocysteine level in the blood, which may lead to spontaneous abortion and pregnancy complications, such as placental abruption and pre-eclampsia Vitamin B12 deficiency can produce atrophic gastritis, pernicious anaemia, thyrotoxicosis; hemorrhage, liver and kidney disease, supplementation with B12 is prescribed after surgical removal of the stomach intestine or gastric bypass. Special populations at risk of deficiencies for B9 or B12 are vegetarians, women during pregnancy, elderly people and babies.

Therefore, consumption of the milk-based products comprising the strains of the invention has health benefit, since production of CLNA will take place when the strains are in contact with LNA. Said LNA may be present in the same milk-based product (such as in a milk infant formula), or can be added as an additional ingredient in another edible product that comprises an amount of the milk-based product and other components.

If the source of LNA is the milk-based product itself, also comprising the strains, it is preferably that this milk-based product be in a dehydrated form in which strains are deactivated. For example in the form of a lyophilized powder. Once rehydrated, for example by re-suspension in a liquid matrix, the bacterial strains become active and they are able to transform the LNA to CLNA.

The scope of the present invention also encompasses bacteria obtained by mutation, variation or recombination of the strains *Lactobacillus oris* ORD0255 (provisional accession number CECT 8240), *Bifidobacterium breve* ORD0123 (provisional accession number CECT 8241), *Bifidobacterium breve* ORD0124 (provisional accession number CECT 8242), *Bifidobacterium breve* ORD0128 (provisional accession number CECT 8239), *Bifidobacterium breve* ORD0134 (provisional accession number CECT 8243), *Bifidobacterium breve* ORD0138 (provisional accession number 8244), *Bifidobacterium breve* ORD0294 (provisional accession number CECT 8246), *Bifidobacterium bifidum* ORD0202 (provisional accession number CECT 8245), provided that the resulting bacteria have the activity of producing CLNA from LNA in a medium comprising at least the fat portion (from animal or vegetal origin) contained in milk-based products, as well as in MRS medium, and with a conversion rate comprised from 30 % to 100 %. In particular embodiments of the invention, the mutant is a genetically modified mutant obtained by classical mutagenesis (i.e. using chemical or physical agents) or by genetic engineering techniques. In another embodiment, the variant is a naturally occurring variant.

The general use of the strains of the invention is in the form of viable cells, although this is not the only way of supplying the strains. When the strains are in the form of viable cells this means the introduction of the living bacteria in a milk-based product, or in an environment comprising a milk-based product. In this way, the administration of such products allows obtaining CLNA if in the media there is also a source of LNA.

As above indicated the present invention provides strains of lactic acid bacteria and bifidobacteria selected from the species of the group consisting of *Bifidobacterium breve*, *Bifidobacterium bifidum,* and *Lactobacillus oris.*

In an embodiment, the invention also relates to mixtures of any of the strains of the invention with another strain of the *Bifidobacterium* genus and/or *Lactobacillus* genus. In a preferred embodiment, the mixture comprises any of the strains of the invention and a strain of the species *Bifidobacterium breve* and/or *Bifidobacterium longum* and/or *Bifidobacterium bifidum* and/or *Bifidobacterium pseudocatenulatum*, and/or *Lactobacillus oris.*

In other embodiments, the strains of the invention may be used for the preparation of a variety of food products, such as a milk products, a yogurt, a curd, a cheese (e.g. quark, cream, processed, soft and hard), a fermented milk, a milk powder, a milk based fermented product, an ice-cream, a fermented cereal based product, a milk based powder, a beverage, a dressing, and a pet food. Examples of other food products are meat products (e.g. liver paste, frankfurter and salami sausages or meat spreads), chocolate spreads, fillings (e.g. truffle, cream) and frostings, chocolate, confectionery (e.g. caramel, fondants or toffee), baked goods (cakes, pastries), sauces and soups, fruit juices and coffee whiteners. However, the term "food product" is used herein in its broadest meaning, including any type of product, in any form of presentation, which can be ingested by an animal, including humans.

In an embodiment, the concentration of LNA in the food product is of at least 0.3 mg/ml or g of the edible (food) composition. In another embodiment, the edible composition comprises 0.5 mg/ml or g of LNA.

In this food product according to the invention, the desired amount of LNA may be provided by the accompanying ingredients in the matrix of the selected food (i.e., in a fat portion of a skimmed cow milked). Alternatively, if the food does not contain LNA as raw ingredient, it can be added to the food composition as a supplement.

In other embodiments, the strains of the invention may be used for the preparation of a variety of nutritional compositions. Particular embodiments are a dietary supplement, an additive, and an infant formula. Dietary supplements intend to supply nutrients (vitamins, minerals, fatty acids or amino acids) that are missing or not consumed in sufficient quantity in a person's diet (infants, pregnant women, elderly people, etc). In a particular embodiment, the strain of the invention is homogenized with other ingredients, such as cereals or powdered milk to constitute an infant formula.

In an embodiment of the nutritional compositions, the concentration of LNA is of at least 0.3 mg/ml or g of the nutritional composition. In another embodiment, the edible composition comprises 0.5 mg/ml or g of LNA.

In such food products and compositions, the strain is present in an amount from about $10^5$ cfu/g to about $10^9$ cfu/g of the composition, and preferably in an amount of $10^7$ cfu/g, according to the current legislation. For the purpose of the present invention the abbreviation "cfu" shall designate a "colony forming unit" that is defined as number of bacterial cells as revealed by microbiological counts on agar plates. Depending on the product, bacteria will be in viable form or non-viable.

The present invention also provides pharmaceutical compositions comprising the strain of the invention, together with pharmaceutical excipients and/or carriers. In this regard, the pharmaceutical composition may be prepared in form of tablets, dried oral supplements, dry tube feeding, etc., with the amount of bacteria to be incorporated therein being in the range of $10^7$ cfu/g to about $10^{11}$ cfu/g of product, and preferably in an amount of $10^9$ cfu/g. Based upon the desired objective the person skilled in the art will select the appropriate excipients and/or carriers. Dried preparations are preferred because they have a better stability.

In an embodiment of the pharmaceutical compositions, the concentration of LNA is of at least 0.3 mg/ml or g of the pharmaceutical composition. In another embodiment, the pharmaceutical composition comprises 0.5 mg/ml or g of LNA.

In general, the compositions of the invention may comprise the bacteria of the invention as single probiotic agent against obesity, combinations of such probiotics or combinations with other therapeutic/nutraceutical agents depending on the condition.

Thus, the invention also provides a strain used in dairy or agroalimentary industry selected from Lactic acid bacteria (LAB) (in particular Lactobacillus) and Bifidobacteria, said strains selected from the species of the group consisting of, *Bifidobacterium breve, Bifidobacterium bifidum*, and *Lactobacillus oris,* wherein the strain is characterized by:

i) being able to grow to an optical density of at least 0.9 $OD_{600}$, determined at 48 hours after initial of growth in anaerobiosis, in a Man Rogosa Sharpe broth medium, as well as in a medium comprising at least the fat portion, from animal or vegetal origin, contained in a milk-based product, both media comprising at least 0.3 mg/ml of linolenic acid in the medium; and

ii) being able to produce conjugated linolenic acid (CLNA) in both media.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

EXAMPLES

Example 1. Production of conjugated fatty acids with selected LAB strains.

A screening was performed with 65 strains of LABORATORIOS ORDESA, S.L., the majority of *Bifidobacterium* genus, in order to analyze the capacity of producing conjugated fatty acids in several media.

The inventors surprisingly found a set of strains which were able to produce high amounts of the conjugated fatty acid CLNA from the corresponding fatty acid, linolenic acid (LNA). The strains were able to provide great conversion rates independently of the tested media. In addition, other conjugated fatty acids were obtained with high yields.

The capacity to produce conjugated linoleic acid (CLA) and conjugated linolenic acid (CLNA) using growing cultures of the strains and 0.5 mg/ml of LA or LNA, was tested in MRS media, and in a in a milk-based medium (milk-based product containing medium). In particular, the strains were tested for its capacity of obtaining the conjugated fatty acids in reconstituted skimmed milk and in an infant formula (Blemil-Plus-1 Forte® of LABORATORIOS ORDESA, S.L.) supplemented with LA and/or with LNA.

Following Table 1.1 shows the optical density at 600 nm (Spectrophotometer Benchmark Plus, Biorad) measured at 37 °C of the mixtures of the strains in MRS supplemented with linoleic acid (LA, 0.5 mg/ml).

Table 1.1 Optical density at 48 H with LA in MRS.

| Strain | OD 600 nm |
|---|---|
| *B. breve* ORD 0123 (provisional CECT 8241) | 1 |
| *B. breve* ORD 0124 (provisional CECT 8242) | 1 |
| *B. breve* ORD 0128 (provisional CECT 8239) | 1.22 |
| *B. breve* ORD 0134 (provisional CECT 8243) | 1.13 |
| *B. breve* ORD 0138 (provisional CECT 8244) | 1.2 |
| *B. breve* ORD 0294 (provisional CECT 8246) | 1.27 |
| *B. bifidum* ORD 0202 (provisional CECT 8245) | 1.09 |
| *L. oris* ORD0255 (provisional CECT 8240) | 1.13 |

Next Table 1.2 shows the optical density at 600 nm (Spectrophotometer Benchmark Plus, Biorad) measured at 37 °C of the mixtures of the strains in MRS supplemented with linolenic acid (LNA, 0.5 mg/ml).

Table 1.2. Optical density at 48 H with LNA in MRS.

| Strain | OD 600 nm |
|---|---|
| *B. breve* ORD 0123 | 3,04 ± 0,4 |
| *B. breve* ORD 0124 | 2,75 ± 0,2 |
| *B. breve* ORD 0128 | 2,83 ± 0,3 |
| *B. breve* ORD 0134 | 3,21 ± 0,5 |
| *B. breve* ORD 0138 | 2,78 ± 0,2 |
| *B. breve* ORD 0294 | 3,78 ± 0,4 |
| *B. bifidum* ORD 0202 | 2,62 ± 0,1 |
| *L. oris* ORD0255 | 3,28 ± 0,3 |

Thus, according to Tables 1.1 and 1.2 all the strains were able to grow to an optical density of at least 0.9 ($OD_{600}$), determined at 48 hours after growing in anaerobiosis, in a Man Rogosa Sharpe broth medium. In particular when LA was in the medium the optical density was comprised from 0.9 $OD_{600}$ to 1.3 $OD_{600}$. On the other hand, when in the medium LNAwas added as ingredient, the optical density was comprised from $OD_{600}$ 2.50 to 4.0 measured at 48 h after growing in anaerobiosis.

Determination and quantification of conjugated fatty acids was done by gas chromatography as described above.

All the reagents and solvents are of laboratory grade purity. Solvents (hexane, methanol, sulphuric acid) from Labscan (Dublin, Ireland). LA form Sigma-Aldrich (St. Louis, MO, USA). LNA from Nu-Chek Prep, Inc. (Elysian, USA).

Methodology for obtaining CLNA:

Bacteria for the screening and other assays realized *a posteriori* were activated in 10 ml of MRS (Pronadisa, Madrid, España) supplemented with 0.25 % (weight/volume; w/v) of L-cysteine (Sigma) and 0.2% (w/v) of Tween-80 (Scharlau, Sentmenat, Barcelona, España). The strains were incubated over-night at 37 °C in anaerobiosis (Anaerobiosis chamber, BactronII from Shellab, Cornelius, Oregon, USA). The day later, strains were reinoculated at 2.5 % at the same conditions with a supplement of LA or LNA at a final concentration of 0.5 mg/ml. The cultures were incubated for 24 hours at 37 °C in anaerobic conditions.

For the analysis of the fatty acids profile, the content of the methyl esters was analyzed by gas chromatography after derivatization of 500 µl of the culture medium by direct transmethylation.

In most of the assays, the FAME content of the global culture medium was analyzed. But a separate analysis of supernatant and pellets in MRS medium was also analyzed (previous separation of the pellet at 10000 r.p.m. for 10 minutes at 4 °C).

Chromatography analysis of CLA and CLNA isomers:

The analysis of the isomers of CLA and CLNA and the total content of the fatty acid methyl esters (FAME) was analyzed by gas chromatography after derivatization of 500 µl of the culture medium by direct transmethylation with $H_2SO_4$/MeOH 10% (w/v) at 95 °C for 30 minutes. Resultant FAMES were analyzed by gas chromatography in a GC-FID (PerkinElmer, Clarus 500) using a column VF-23 (Varian). The chromatographic program was started at 80 °C and after a rate of 30 °C/min until 230°C and maintenance of the temperature for 7 min was employed. Helium (He) was used as a carrier gas and the pressure was of 15 psi.

In all cases, for the identification and quantification of the different methyl esters, pure fatty acids as standards were used, as well as oils and fats of known composition. C17-heptadecanoic acid was also used as internal standard.

As positive control a *Bifidobacterium breve* 26M2 strain was used, known to be able to produce CLA and CLNA in MRS. All the assays were performed in triplicate. The strain *Bifidobacterium breve* 26M2 is the one identified in Jiménez E, et al., "Complete genome sequence of Bifidobacterium breve CECT 7263, a strain isolated from human milk", J Bacteriol. 2012, Vol. 194(14), pp.:3762-3.

Table 2 shows the list of strains selected for being able to transform LNA (and LA) to CLNA (and CLA) in the three different tested media. In this table it is also indicated other features of the strains or other compounds obtainable with the method of the invention being performed with bacterial strains. The concentration of CLA and CLNA obtained in each media is also included.

All these selected strains were deposited in the Colección Española de Cultivos Tipo (CECT) under the Depositor’s reference and provisional accession numbers indicated also in the Table 2. In Example 2 there are listed other features of the strains as well as their isolation mode.

Table 2. Results of the method.

| Strain | CLA *(µg/mL) in MRS* | CLA *(µg/mL) in RSM* | CLA *(µg/mL) in Blemil-Plus-1 Forte®* | CLNA *(µg/mL) in MRS* | CLNA *(µg/mL) in RSM* | CLNA *(µg/mL) in Blemil-Plus-1 Forte®* | Other |
|---|---|---|---|---|---|---|---|
| *Bifidobacterium breve* ORD 0123 (provisional CECT 8241) | 108,8 ± 28,9 | 104,9 ± 11,6 | 55,9 ± 4,9 | 209 ± 56,2 | 211,3 ± 62,8 | 193,7 ± 33,8 | Vitamins B12 (0.11 µg/100 ml) and B9 (0.40 µg/100 ml) |
| *Bifidobacterium breve* ORD 0124 (provisional CECT 8242) | 135,1 ± 7,4 | 106,3 ± 25,2 | 65,7 ± 2,6 | 197,8 ± 50,7 | 244,5 ± 43,5 | 201,3 ± 30,6 | |
| *Bifidobacterium breve* ORD 0128 (provisional CECT 8239) | 142,4 ± 8,3 | 98,5 ± 21 | 59,5 ± 3,9 | 208,9 ± 72,0 | 244,9 ± 46,5 | 199,2 ± 17,1 | |
| *Bifidobacterium breve* ORD 0134 (provisional CECT 8243) | 148,1 ± 13,0 | 98,4 ± 16,1 | 54,7 ± 3,5 | 198 ± 53,1 | 230,5 ± 34,7 | 177,9 ± 25,2 | Vitamin B9 (0.36 µg/100 ml) |
| | | | | | | | |
| *Bifidobacterium breve* ORD 0138 (provisional CECT 8244) | 163 ± 30,9 | 98,1 ± 9,3 | 64,0 ± 4,2 | 201,8 ± 50,9 | 237,5 ± 23 | 199,7 ± 34,9 | |

(continued)

| Strain | CLA ($\mu$g/mL) in MRS | CLA ($\mu$g/mL) in RSM | CLA ($\mu$g/mL) in Blemil-Plus-1 Forte® | CLNA ($\mu$g/mL) in MRS | CLNA ($\mu$g/mL) in RSM | CLNA ($\mu$g/mL) in Blemil-Plus-1 Forte® | Other |
|---|---|---|---|---|---|---|---|
| *Bifidobacterium breve* ORD 0294 (provisional CECT 8246) | 148,5 ± 7,9 | 92 ± 23,2 | 56,8 ± 7,1 | 190,7 ± 53 | 243,6 ± 39,8 | 177,8 ± 19,1 | Vitamin B12 (0.12 $\mu$g/100 ml) |
| *Bifidobacterium bifidum* ORD 0202 (provisional CECT 8245) | 113,9 ± 9,9 | 18,5 ± 13,3 | 32,6 ± 3,6 | 217,9 ± 86,1 | 220,5 ± 38,7 | 85,7 ± 41,6 | |
| *Lactobacillus oris* ORD 0255 (provisional CECT 8240) | 78,9 ± 11,8 | 98.5 ± 27,7 | 20,8 ± 4,2 | 191,3 ± 66,4 | 241,6 ± 44,0 | 81,1 ± 18,3 | Vitamin B9 (0.21 $\mu$g/100 ml) |

Production of Vitamins B12 and B9 (folic acid) in the selected strains were measured using a commercial system, VitaFast Vitamin B12 (Cyanocobalamin) kit and VitaFast® Folic Acid (R-Biopharm, Darmstadt, Germany) according to manufacturer's instructions.

Of special interest are those strains that a part of being able to produce great amounts of CLNA, they were also producers of vitamins and great producer of the other conjugated fatty acid CLA.

The results of the assays performed in RSM and in the infant formula are, moreover, illustrated in FIGs. 1 to 3.

FIG. 1 shows the percentage of conversion of LNA to CLNA when the method of the invention is performed in the presence of each strain and using a medium comprising RSM as the milk-based product. Numbers over the bars indicate the value of the calculated conversion rate.

As can be seen in this FIG. 1 the percentages of conversion (conversion rates) were all near 100 % in the strains of the species *Bifidobacterium breve, Bifidobacterium breve* and *Lactobacillus oris.* That is, a conversion rate comprised from 30 % to 100 %. In particular from 60 % to 99 %.

From each sample (assay), the identification of the isomers of CLNA was determined as indicated in the methodology. FIG. 2 shows the percentage by weight of the isomer C18:3 *cis*-9 *trans*-11 *cis*-15 in relation with the total weight of CLNA obtained and indicated in Table 2.

On the other hand, the results of the method applied in a medium comprising an infant formula are depicted in FIG. 3.

FIG. 3 shows the concentration of CLNA ($\mu$g/ml) obtained as a mean of the three assays performed with each strain. Numbers over the bars indicate the concentration obtained for each strain (and correspond with the ones in Table 2).

The obtained concentrations were comprised from 81.1 to 201.3 $\mu$g/ml. The conversion rates (not shown) were comprised from 30 % to 80 %. In particular, all the strains showed a conversion rate of 75 %, except of the methods performed with strains *Lactobacillus oris* ORD0255 (provisional CECT 8240), and *Bifidobacterium bifidum* OR0D202 (provisional CECT 8245), in which a conversion rate of 33.7 % and 36.9 % were obtained.

<u>Example 2.</u> Isolation and characterization of lactic acid bacteria (LAB) (Lactobacillus) and Bifidobacteria.

<u>2.1. Isolation of microorganisms</u>

In the case of Bacteria isolated from infant feces of breast fed infant, 0.5 g of feces was dissolved in PBS buffer (phosphate buffer pH 7.0) homogenizing the sample by means of maceration during 10 min in a stomacher (Stomacher 80 Biomaster, Seward, UK). In the case of Bacteria isolated from breastmilk, 0.5 ml of breastmilk was also dissolved in PBS buffer homogenizing the sample by means of maceration during 10 min in a stomacher. A 1 ml aliquot of the homogenate was serial diluted in PBS and 0.1 ml aliquots of different dilutions were seeded in plates of several culture media including Man Rogosa Sharpe agar (MRS) supplemented with or without 0.25% (w/v) of cysteine (MRS-C; Sigma, St. Louis, Mo.) other culture media used were selective medium for *Bifidobacterium* (BSM, Fluka, Buchs, Switzerland) and *Bifidobacterium* medium agar (BFM; Y. Nebra et al., "A new selective medium for Bifidobacterium spp." Applied and Enviromental Microbiology, 1999, vol. 65, pp. 5173-6), Brain Heart Infusion agar, Agar Tomato, Rogosa agar and were incubated during 48 h. Morphology of the colony and the type of cellular wall of the obtained strains were determined by Gram staining.

Some strains were selected for characterization. The following results are for the selected strains of the invention, bacteria were deposited in the Colección Española de Cultivos Tipo (CECT) under the Depositor's references *Bifidobacterium bifidum* ORD0202 (provisional accession number CECT 8245), *Bifidobacterium breve* ORD0123 (provisional accession number CECT 8241), *Bifidobacterium breve* ORD0124 (provisional accession number CECT 8242), *Bifidobacterium breve* ORD0128 (provisional accession number CECT 8239), *Bifidobacterium breve* ORD0134 (provisional accession number CECT 8243), *Bifidobacterium breve* ORD0138 (provisional accession number CECT 8244), *Bifidobacterium breve* ORD0294 (provisional accession number CECT 8246) have been isolated from babies feces and *Lactobacillus oris* ORD0255 (provisional accession number CECT 8240) from human breastmilk.

2.2 Taxonomic characterization of strains by sequencing

Almost full sequence of the 16S rRNA gene was amplified and sequenced using a ABI PRISM BigDye Terminator Cycle Sequencing Ready Reaction Kit. The DNA was checked for purity, using standard methods.

DNA templates were amplified by the polymerase chain reaction (PCR) on a thermocycler, using universal primers amplifying a 348 bp region of the 16S rRNA gene, Y1: 5'-TGG CTC AGG ACG AAC GCT GGC GGC-3' (SEQ ID NO: 9), Y2: 5'-CCT ACT GCT GCC TCC CGT AGG AGT-3' (SEQ ID NO: 10), and a 1,004 bp region of the 16S rRNA gene, 16s1 a: 5'-AAT ACA TGC AAG TCG AAC GA-3' (SEQ ID NO: 11), 16s1 b: 5'-TTA ACC CAA CAT CTC ACG AC-3' (SEQ ID NO: 12). The amplification mixture (50 μl) comprised 1.5μl (100 pmol/μl) ofeach of Y1 and Y2 primers or 16s1 a and 16s1 b, 1 μl (1U/μl) of Taq DNA Polymerase KOD Hot Start (Merck), 5 μl of 10x KOD Hot Start buffer (Merck), 5 μl of dNTP mixture (containing 1 mM each of dATP, dGTP, dCTP and dTTP, Merck), 3 μl of 25mM $MgSO_4$ (Merck), 31 μl of sterile filtered water (Milli-Q purification system, Millipore) and 2 μl of DNA template. The DNA templates were amplified using Y1 and Y2 primers by initial denaturation at 95° C for 3 min, followed by 30 cycles of denaturation at 95° C for 2.3 min, annealing at 70° C for 30 sec, extension at 70° C for 40 sec, and a final extension at 70° C for 7 min. The DNA templates were amplified using 16s1 a and 16s1 b oligos by initial denaturation at 95° C for 2 min, followed by 30 cycles of denaturation at 95° C for 1 min, annealing at 61° C for 30 sec, extension at 72° C for 1.30 min, and a final extension at 72° C for 10 min. Controls, devoid of DNA, were simultaneously included in the amplification process. The integrity of PCR products was assayed by the development of single bands following electrophoresis for 1 h at 100 V in 2% or 1% (w/v) agarose gels in tris-borate EDTA buffer.

Amplicons were purified using a commercial kit and subsequent sequencing reactions were performed using the Big Dye Terminator v3.1 cycle sequencing kit, premixed format. Sequencing primers were the same ones used in the amplification reaction but diluted ten times (5 pmol).

The resulting sequences were automatically aligned for each strain and then inspected by eye. The resulting 16S rRNA gene sequences were compared in a BLAST search with those in the National Library of Medicine database.

The following sequences obtained SEQ ID NO:1 to SEQ ID NO:8 correspond to the 16S rRNA of any of the strains, amplified with primers of sequences SEQ ID NO: 11 and 12, and they correlate with the identification of the strains as follows:

SEQ ID NO:1 correlates with *Bifidobacterium breve* ORD0123 (provisional accession number CECT 8241):

CTTTGCGGCATGGGATGGGGTCGCGTCCTATCAGCTTGATGGCGGGGT
AACGGCCCACCATGGCTTCGACGGGTAGCCGGCCTGAGAGGGCGACCG
GCCACATTGGGACTGAGATACGGCCCAGACTCCTACGGGAGGCAGCAG
TGGGGAATATTGCACAATGGGCGCAAGCCTGATGCAGCGACGCCGCGT
GAGGGATGGAGGCCTTCGGGTTGTAAACCTCTTTTGTTAGGGAGCAAGG
CATTTTTTGTTGA

SEQ ID NO:2 correlates with *Bifidobacterium breve* ORD0124 (provisional accession number CECT 8242):

AATAGCTCCTGGAAACGGGTGGTAATGCCGGATGCTCCATCACACCGCA
TGGTGTGTTGGGAAAGCCTTTGCGGCATGGGATGGGGTCGCGTCCTATC
AGCTTGATGGCGGGGTAACGGCCCACCATGGCTTCGACGGGTAGCCGG
CCTGAGAGGGCGACCGGCCACATTGGGACTGAGATACGGCCCAGACTC
CTACGGGAGGCAGCAGTGGGGAATATTGCACAATGGGCGCAAGCCTGA
TGCAGCGACGCCGCGTGAGGGATGGAGGCCTTCGGGTTGTAAACCTCT
TTTGTTAGGGAGCAAGGCATTTTTTGTTGAGTGTACCTTTCGAATAAGCA
CCGGCTAACTACGTGCCAGCAGCCGCGGTAATACGTAGGGTGCAAGCG
TTATCCGGAATTATTGGGCGTAAAGGGCTCGTAGGCGGTTCGTCGCGTC
CGGTGTGAAAGTCCATCGCTTAACGGTGGATCCGCGCCGGGTACGGGC
GGGCTTGAGTGCGGTAGGGGAGACTGGAATTCCCGGTGTAACGGTGGA
ATGTGTAGATATCGGGAAGAACACCAATGGCGAAGGCAGGTCTCTGGGC

CGTTACTGACGCTGAGGAGCGAAAGCGTGGGGAGCGAACAGGATTAGA
TACCCTGGTAGTCCACGCCGTA

SEQ ID NO:3 correlates with *Bifidobacterium breve* ORD0128 (provisional accession number CECT 8239):

GATGGCGGGGTAACGGCCCACCATGGCTTCGACGGGTAGCCGGCCTGA
GAGGGCGACCGGCCACATTGGGACTGAGATACGGCCCAGACTCCTACG
GGAGGCAGCAGTGGGGAATATTGCACAATGGGCGCAAGCCTGATGCAG
CGACGCCGCGTGAGGGATGGAGGCCTTCGGGTTGTAAACCTCTTTTGTT
AGGGAGCAAGGCATTTTTTGTTGAGTGTACCTTTCGAATAAGCACCGGCT
AACTACGTGCCAGCAGCCGCGGTAATACGTAGGGTGCAAGCGTTATCCG
GAATTATTGGGCGTAAAGGGCTCGTAGGCGGTTCGTCGCGTCCGGTGT
GAAAGTCCATCGCTTAACGGTGGATCCGCGCCGGGTACGGGCGGGCTT
GAGTGCGGTAGGGGAGACTGGAATTCCCGGTGTAACGGTGGAATGTGT
AGATATCGGGAAGAACACCAATGGCGAAGGCAGGTCTCTGGGCCGTTAC
TGACGCTGAGGAGCGAAAGCGTGGGGAGCGAACAGGATTAGATACCCT
GGTAGTCCACGCCGTAAACGGTGGATGCTGGATGTGGGGCCCGTTCCA
CGGGTTCCGTGTCGGAGCTAACGCGTTAAGCATCCCGCCTGGGGAGTA
CGGCCGCAAGGCTAAAACTCAAAGAAATTGACGGGGGCCCGCACAAGC
GGCGGAGCATGCGGATTAATTCGATGCAACGCGAAGAACCTTACCTGGG
CTTGACATGTTCCCGACGATCCCAGAGATGGGGTT

SEQ ID NO:4 correlates with *Bifidobacterium breve* ORD0134 (provisional accession number CECT 8243):

GCGGCATGGGATGGGGTCGCGTCCTATCAGCTTGATGGCGGGGTAACG
GCCCACCATGGCTTCGACGGGTAGCCGGCCTGAGAGGGCGACCGGCCA
CATTGGGACTGAGATACGGCCCAGACTCCTACGGGAGGCAGCAGTGGG
GAATATTGCACAATGGGCGCAAGCCTGATGCAGCGACGCCGCGTGAGG
GATGGAGGCCTTCGGGTTGTAAACCTCTTTTGTTAGGGAGCAAGGCATT
TTTTGTTGAGTGTACCTTTCGAATAAGCACCGGCTAACTACGTGCCAGCA
GCCGCGGTAATACGTAGGGTGCAAGCGTTATCCGGAATTATTGGGCGTA
AAGGGCTCGTAGGCGGTTCGTCGCGTCCGGTGTGAAAGTCCATCGCTTA
ACGGTGGATCCGCGCCGGGTACGGGCGGGCTTGAGTGCGGTAGGGGA
GACTGGAATTCCCGGTGTAACGGTGGAATGTGTAGATATCGGGAAGAAC
ACCAATGGCGAAGGCAGGTCTCTGGGCCGTTACTGACGCTGAGGAGCG

AAAGCGTGGGGAGCGAACAGGATTAGATACCCTGGTAGTCCACGCCGTA
AACGGTGGATGCTGGATGTGGGGCCCGTTCCACGGGTTCCGTGTCGGA
GCTAACGCGTTAAGCATCCCGCCTGGGGAGTACGGCCGCAAGGCTAAAA
CTCAAAGAAATTGACGGGGGCCCGCACAAGCGGCGGA

SEQ ID NO:5 correlates with *Bifidobacterium breve* ORD0138 (provisional accession number CECT 8244):

TTGCGGCATGGGATGGGGTCGCGTCCTATCAGCTTGATGGCGGGGTAA
CGGCCCACCATGGCTTCGACGGGTAGCCGGCCTGAGAGGGCGACCGG
CCACATTGGGACTGAGATACGGCCCAGACTCCTACGGGAGGCAGCAGT
GGGGAATATTGCACAATGGGCGCAAGCCTGATGCAGCGACGCCGCGTG
AGGGATGGAGGCCTTCGGGTTGTAAACCTCTTTTGTTAGGGAGCAAGGC
ATTTTTTGTTGAGTGTACCTTTCGAATAAGCACCGGCTAACTACGTGCCA
GCAGCCGCGGTAATACGTAGGGTGCAAGCGTTATCCGGAATTATTGGGC
GTAAAGGGCTCGTAGGCGGTTCGTCGCGTCCGGTGTGAAAGTCCATCG
CTTAACGGTGGATCCGCGCCGGGTACGGGCGGGCTTGAGTGCGGTAGG
GGAGACTGGAATTCCCGGTGTAACGGTGGAATGTGTAGATATCGGGAAG
AACACCAATGGC

SEQ ID NO:6 correlates with *Bifidobacterium bifidum* ORD0202 (provisional accession number CECT 8245):

TCCTGGAAACGGGTGGTAATGCCGGATGTTCCACATGATCGCATGTGAT
TGTGGGAAAGATTCTATCGGCGTGGGATGGGGTCGCGTCCTATCAGCTT
GTTGGTGAGGTAACGGCTCACCAAGGCTTCGACGGGTAGCCGGCCTGA
GAGGGCGACCGGCCACATTGGGACTGAGATACGGCCCAGACTCCTACG
GGAGGCAGCAGTGGGGAATATTGCACAATGGGCGCAAGCCTGATGCAG
CGACGCCGCGTGAGGGATGGAGGCCTTCGGGTTGTAAACCTCTTTTGTT
TGGGAGCAAGCCTTCGGGTGAGTGTACCTTTCGAATAAGCGCCGGCTAA
CTACGTGCCAGCAGCCGCGGTAATACGTAGGGCGCAAGCGTTATCCGG
ATTTATTGGGCGTAAAGGGCTCGTAGGCGGCTCGTC

SEQ ID NO:7 correlates with *Lactobacillus oris* ORD0255 (provisional CECT accession number 8240):

GGGGATAACATTTGGAAACAGGTGCTAATACCGCATAACTTGGAAAACCA

CATGGTTTTCCAATAAAAGATGGTTTCGGCTATCACTTTGGGATGGGCCC
GCGGTGCATTAGCTAGTTGGTAAGGTAACGGCTTACCAAGGCGATGATG
CATAGCCGAGTTGAGAGACTGATCGGCCACAATGGAACTGAGACACGGT
CCATACTCCTACGGGAGGCAGCAGTAGGGAATCTTCCACAATGGGCGCA
AGCCTGATGGAGCAACACCGCGTGAGTGAAGAAGGGTTTCGGCTCGTAA
AACTCTGTTGTTGGAGAAGAACGTGCGTAAGAGTAACTGTTTACGCAGTG
ACGGTATCCAACCAGAAAGTCACGGCTAACTACGTGCCAGCAGCCGCGG
TAATACGTAGGTGGCAAGCGTTATCCGGATTTATTGGGCGTAAAGCGAG
CGCAGGCGGTTGCTTAGGTCTGATGTGAAAGCCTTCGGCTTAACCGAAG
AAGTGCATCGGAAACCGGGCGACTTGAGTGCAGAAGAGGACAGTGGAA
CTCCATGTGTAGCGGTGGAATGCGTAGATATATGGAAGAACACCAGTGG
CGAAGGCGGCTGTCTGGTCTGCAACTGACGCTGAGGCTCGAAAGCATG
GGTAGCGAACAGGATTAGATACCCTGGTAGTCCATGCCGTAAACGATGA
GTGCTAGGTGTTGGAGGGTTTCCGCCCTTCAGTGCCGAAGCTAACGCAT
TAAGCACTCCGCCTGGGGAGTACGACCGCAAGGTTGAAACT

SEQ ID NO:8 correlates with *Bifidobacterium breve* ORD0294 (provisional accession number CECT 8246):

GCGGCATGGGATGGGGTCGCGTCCTATCAGCTTGATGGCGGGGTAACG
GCCCACCATGGCTTCGACGGGTAGCCGGCCTGAGAGGGCGACCGGCCA
CATTGGGACTGAGATACGGCCCAGACTCCTACGGGAGGCAGCAGTGGG
GAATATTGCACAATGGGCGCAAGCCTGATGCAGCGACGCCGCGTGAGG
GATGGAGGCCTTCGGGTTGTAAACCTCTTTTGTTAGGGAGCAAGGCATT
TTTTGTTGAGTGTACCTTTCGAATAAGCACCGGCTAACTACGTGCCAGCA
GCCGCGGTAATACGTAGGGTGCAAGCGTTATCCGGAATTATTGGGCGTA
AAGGGCTCGTAGGCGGTTCGTCGCGTCCGGTGTGAAAGTCCATCGCTTA
ACGGTGGATCCGCGCCGGGTACGGGCGGGCTTGAGTGCGGTAGGGGA
GACTGGAATTCCCGGTGTAACGGTGGAATGTGTAGATATCGGGAAGAAC
ACCAATGG

Following, with the semi-quantitative method API ZYM®, it was determined the enzymatic activity of all these strains sharing the capability of transforming LNA to CLNA independently of the culture media.

Table 3 shows the results obtained for each selected strain.

Table 3. Enzymatic profile of selected strains

| Strain | Control | Alkaline Phosphatase | Esterase (C4) | Esterase Lipase (C8) | Lipase (C14) | Leucine arylamidase |
|---|---|---|---|---|---|---|
| ORD 0123 | 0 | 0 | 1 | 0 | 0 | 5 |
| ORD 0124 | 0 | 0 | 1 | 1 | 0 | 5 |
| ORD 0128 | 0 | 0 | 1 | 2 | 0 | 5 |
| ORD 0134 | 0 | 0 | 1 | 1 | 0 | 5 |
| ORD 0138 | 0 | 0 | 1 | 1 | 0 | 5 |
| ORD 0202 | 0 | 2 | 1 | 0 | 0 | 5 |
| ORD 0255 | 0 | 3 | 2 | 2 | 0 | 5 |
| ORD 0294 | 0 | 0 | 3 | 2 | 0 | 5 |

| Strain | Valine arylamidase | Cistine arylamidase | Trypsin | α-chemotrypsin | Acid Phosphatase | Naftol-AS-BI-(+) | α-galactosidase | β-galactosidase |
|---|---|---|---|---|---|---|---|---|
| ORD 0123 | 1 | 1 | 0 | 0 | 4 | 0 | 3 | 5 |
| ORD 0124 | 1 | 1 | 0 | 0 | 3 | 0 | 2 | 5 |
| ORD 0128 | 1 | 1 | 0 | 0 | 1 | 0 | 4 | 5 |
| ORD 0134 | 1 | 3 | 0 | 0 | 2 | 0 | 3 | 5 |
| ORD 0138 | 1 | 3 | 0 | 0 | 2 | 1 | 4 | 5 |
| ORD 0202 | 1 | 3 | 0 | 0 | 2 | 1 | 4 | 5 |
| ORD 0255 | 2 | 3 | 0 | 0 | 2 | 1 | 4 | 5 |
| ORD 0294 | 1 | 1 | 0 | 0 | 2 | 0 | 0 | 5 |

(+)Naftol-AS-BI-phosphohydrolase

| Strain | β-glucoronidase | α-glucosidase | β-glucosidase | N-acetil-β-glucosaminidase | α-mannosidase | α-fucosidase |
|---|---|---|---|---|---|---|
| ORD 0123 | 0 | 5 | 5 | 0 | 3 | 0 |
| ORD 0124 | 0 | 5 | 5 | 0 | 0 | 0 |
| ORD 0128 | 0 | 5 | 5 | 1 | 0 | 0 |
| ORD 0134 | 0 | 5 | 4 | 0 | 0 | 0 |
| ORD 0138 | 0 | 5 | 5 | 0 | 0 | 0 |
| ORD 0202 | 0 | 5 | 4 | 0 | 0 | 0 |
| ORD 0255 | 0 | 5 | 5 | 0 | 0 | 0 |
| ORD 0294 | 0 | 0 | 0 | 0 | 0 | 0 |

As can be deduced from this Table 3, all of the strains were positive (presence of enzymatic activity) for Leucine arilamidase (+) and β-galactosidase (+). On the other hand all of them were negative (absence of enzymatic activity) for Lipase (-), Trypsin (-), Chemotrypsin (-), and β-glucuronidase (-).

As a comparative example is to be said that a strain of *Bifidobacterium animalis,* which had a different enzymatic profile than the selected strains, had not the ability to produce CLA or CLNA from LA or LNA when was grown in MRS, RSM or infant formula.

Example 3·Efffect of the selected strains on *Caenorhabditis elegans (C. elegans)* of diets comprising the strains of the invention.

*C. elegans* strain N2, Bristol (wild-type), was obtained from the *Caenorhabditis* Genetics Centre (CGC) at the University of Minnesota (USA) and maintained at 20°C on nematode growth medium (NGM). *Escherichia coli* OP50 strain was used as normal nematode diet and was also provided by the CGC. Worms were grown on NGM using *E. coli* OP50 as control diet

The effects of CLNA and CLA strains producers on body-fat reduction in C. *elegans* N2 were studied by measuring fluorescence of stained worms. Populations of age-synchronized worms were obtained by isolating eggs from gravid adults and hatching the eggs overnight in NGM plates (as control media), NGM plates with 6 μg/mL of Orlistat (Sigma-Aldrich, Madrid, Spain) used as positive control and NGM plates supplemented with the different strains. Nile Red (9-diethylamino-5H-benzo[α]phenoxazin-5-one, Sigma, St. Louis, MO, USA) was used as dye to monitor lipid storage in live worms. The dye was added on the top of the NGM agar plates, preseeded with Escherichia coli OP50, to a final concentration of 0.05 μg/mL. Worms were incubated at 20°C for 3 days until young adult stage. After this incubation period, nematode samples were placed in M9 buffer and fluorescence was measured in an FP-6200 system (JASCO Analytical Instruments, Easton, MD, USA) using λ excitation 480 nm and λ emission 571 nm. A total of 90 worms per condition were analyzed (Martorell et al., 2012 *supra*). Experiments were carried out in triplicate. Results were represented as reduction of fluorescence versus control.

Next Table 4, indicates the percentage (%) of fluorescence reductions in C. *elegans* fed with each strain. The p-value obtained in statistical analysis (ANOVA test) is also showed. NS: means No Statistical Significance. In this model % of fluorescence reduction is correlated with reduction of adipogenesis in *C. elegans.*

Table 4.

| Samples | % Reduction Fluorescence | p-value |
|---|---|---|
| NGM | 0 | |
| NGM + Orlistat (6μg/mL) | 30,32 ± 2,5 | ≤ 0,001 |
| NGM + *B. breve* ORD0123 (provisional CECT 8241) | 9,74 ± 2,4 | ≤ 0,01 |
| NGM + *B. breve* ORD0124 (provisional CECT 8242) | 27,12 ± 1,4 | ≤ 0,001 |
| NGM + *B. breve* ORD0128 (provisional CECT 8239) | 9,09 ± 1,19 | ≤ 0,01 |
| NGM + *B. breve* ORD0134 (provisional CECT 8243) | 16,34 ± 4,73 | ≤ 0,001 |
| NGM + *B. breve* ORD0138 (provisional CECT 8244) | 2,04 ± 3,54 | NS |
| NGM + *B. bifidum* ORD0202 (provisional CECT 8245) | 12,48 ± 2,8 | ≤ 0,001 |
| NGM + *L. oris* ORD0255 (provisional CECT 8240) | 28,36 ± 2,59 | ≤ 0,001 |
| NGM + *B. breve* ORD0294 (provisional CECT 8246) | 5,79 ± 6,5 | ≤ 0,05 |

As can be deduced from the data in the Table 4, all of the strains promoted reduction of fat storing in the *in vivo* model *C. elegans.* In addition, the diet including *L. oris* ORD0255 (provisional accession number CECT 8240), and the diet including *B. breve* ORD0124 (provisional accession number CECT 8242) showed reduction percentages near to the one of the positive control (Orlistat). Thus, all these data allow concluding that the strains producers of CLNA and CLA in all the tested media are potential anti-obesity agents.

This property is highly interesting much more because the strains can be supplied in an edible product (infant formula in particular) aimed to control or prevent obesity development in children and babies.

REFERENCES CITED IN THE APPLICATION

- Gorissen et al, "Production of conjugated linoleic and linolenic acid isomers by Bifidobacterium species", Appl. Microbiol Biotechnol - 2010, vol. 87, pp.: 2257-2266
- Hennessy et al., "The Production of Conjugated α-Linolenic, γ-Linolenic and Stearidonic Acids by Strains of Bifidobacteria and Propionibacteria", Lipids - online published 10 Dec 2011, DOI 10.1007/s11745-011-3636-z.
- Gorissen et al., "Microbial production of conjugated linoleic and linolenic acids in fermented foods: Technological bottlenecks", Eur. J. Lipid Sci. Technol. - 2012, DOI: 10.1002/ejlt.201100239.
- Miranda et al., "cis-9, trans-11, cis-15 and cis-9, trans-13, cis-15 CLNA mixtures activates PPARα in HEK293 and reduces triacylglycerols in 3T3-L1 cells", Lipids -2011, vol. 46(11), pp.: 1006-1012.
- Miranda J et al., "cis-9,trans-11 ,cis-15 and cis-9,trans-13,cis-15 CLNA mixture activates PPARα in HEK293 and reduces triacylglycerols in 3T3-L1 cells", Lipids. - 2011, Nov;46(11):1005-12. Epub 2011 Oct 9.
- Miranda J et al., "A comparison between CLNA and CLA effects on body fat, serum parameters and liver composition", J Physiol Biochem.
- 2009, Mar;65(1):25-32.
- Martorell P et al., "Caenorhabditis elegans as a Model To Study the Effectiveness and Metabolic Targets of Dietary Supplements Used for Obesity Treatment: The Specific Case of a Conjugated Linoleic Acid Mixture (Tonalin)", J Agric Food Chem - 2012 Nov 7;60(44):11071-9.
- Jiménez E, et al., "Complete genome sequence of Bifidobacterium breve CECT 7263, a strain isolated from human milk", J Bacteriol. 2012, Vol. 194(14), pp.:3762-3.

## Claims

**1.** A method for preparing an isomer of conjugated linolenic acid (CLNA) from linolenic acid (LNA) in a medium comprising at least the fat portion from animal or vegetal origin contained in a milk-based product, said fat portion containing linolenic acid, the method comprising adding to the medium at least one strain used in dairy or agroalimentary industry, selected from the group consisting of a lactic acid bacteria (LAB) and a Bifidobacteria, said strain **characterized by**:

i) being able to grow to an optical density of at least 0.9 $OD_{600}$, determined at 48 hours after initial of growth in anaerobiosis, in a Man Rogosa Sharpe broth medium, as well as in a medium comprising at least the fat portion, from animal or vegetal origin, contained in a milk-based product, both media comprising at least 0.3 mg/ml of linolenic acid in the medium; and
ii) being able to produce conjugated linolenic acid (CLNA) in both media.

**2.** The method of claim 2, wherein the conjugated linolenic acid obtained is the isomer C18:3 *cis*-9 *trans*-11 *cis*-15 conjugated linolenic acid.

**3.** The method according to any of the claims 1-2, wherein the strain used in dairy or agroalimentary industry, selected from the group consisting of a lactic acid bacteria (LAB) and Bifidobacteria, is a strain of the bacterial species selected from the group consisting of *Bifidobacterium breve, Bifidobacterium bifidum,* and *Lactobacillus oris.*

**4.** The method according to any of the claims 1-3, wherein the strain is selected from the group consisting of:

- *Bifidobacterium breve* deposited in the Colección Española de Cultivos Tipo (CECT) under the Depositor's reference ORD0123, or a mutant or variant thereof;
- *Bifidobacterium breve* deposited in the Colección Española de Cultivos Tipo (CECT) under the Depositor's reference ORD0124, or a mutant or variant thereof;
- *Bifidobacterium breve* deposited in the Colección Española de Cultivos Tipo (CECT) under the Depositor's reference ORD0128, or a mutant or variant thereof;
- *Bifidobacterium breve* deposited in the Colección Española de Cultivos Tipo (CECT) under the Depositor's reference ORD0134, or a mutant or variant thereof;
- *Bifidobacterium breve* deposited in the Colección Española de Cultivos Tipo (CECT) under the Depositor's reference ORD0138, or a mutant or variant thereof;
- *Bifidobacterium breve* deposited in the Colección Española de Cultivos Tipo (CECT) under the Depositor's reference ORD0294, or a mutant or variant thereof;
- *Bifidobacterium bifidum* deposited in the Colección Española de Cultivos Tipo (CECT) under the Depositor's reference ORD202, or a mutant or variant thereof; and
- *Lactobacillus oris* deposited in the Colección Española de Cultivos Tipo (CECT) under the Depositor's reference ORD255, or a mutant or variant thereof,

wherein the mutants or variants of said strains are obtained using one of the deposited strains as starting material, and wherein the mutant strains retain the essential properties of the deposited strains, wherein said essential properties are:

i) being able to grow to an optical density of at least 0.9 $OD_{600}$, determined at 48 hours after initial of growth in anaerobiosis, in a Man Rogosa Sharpe broth medium, as well as in a medium comprising at least the fat portion, from animal or vegetal origin, contained in a milk-based product, both media comprising at least 0.3 mg/ml of linolenic acid in the medium; and
ii) being able to produce conjugated linolenic acid (CLNA) in both media.

**5.** A Bifidobacteria strain, which is a strain of *Bifidobacterium breve* selected from the group consisting of:

- *Bifidobacterium breve* deposited in the Colección Española de Cultivos Tipo (CECT) under the Depositor's reference ORD0123, or a mutant or variant thereof;
- *Bifidobacterium breve* deposited in the Colección Española de Cultivos Tipo (CECT) under the Depositor's reference ORD0124, or a mutant or variant thereof;
- *Bifidobacterium breve* deposited in the Colección Española de Cultivos Tipo (CECT) under the Depositor's reference ORD0128, or a mutant or variant thereof;
- *Bifidobacterium breve* deposited in the Colección Española de Cultivos Tipo (CECT) under the Depositor's reference ORD0134, or a mutant or variant thereof;
- *Bifidobacterium breve* deposited in the Colección Española de Cultivos Tipo (CECT) under the Depositor's reference ORD0138, or a mutant or variant thereof;
- *Bifidobacterium breve* deposited in the Colección Española de Cultivos Tipo (CECT) under the Depositor's reference ORD0294, or a mutant or variant thereof;
- *Bifidobacterium bifidum* deposited in the Colección Española de Cultivos Tipo (CECT) under the Depositor's reference ORD202, or a mutant or variant thereof.

**6.** A Bifidobacteria strain, which is a strain of *Bifidobacterium bifidum* deposited in the Colección Española de Cultivos Tipo (CECT) under the under the Depositor's reference ORD0202, or a mutant or variant thereof.

**7.** A Lactic acid bacteria strain, which is a strain of *Lactobacillus oris* deposited in the Colección Española de Cultivos Tipo (CECT) under the under the Depositor's reference ORD0255, or a mutant or variant thereof.

**8.** A food product which comprises an effective amount of at least one of the strains as defined in any of the claims 5-7 and LNA as active ingredients, together with appropriate amounts of other edible ingredients or carriers.

**9.** A nutritional composition which comprises a nutritionally effective amount of at least one of the strains as defined in any of the claims 5-7, and LNA as active ingredients, together with appropriate amounts of other appropriate edible ingredients or carriers.

**10.** The nutritional composition according to claim 9, which is selected from the group consisting of a dietary supplement, an additive and an infant formula.

**11.** A pharmaceutical composition which comprises a therapeutically effective amount of at least one of the strains as defined in any of the claims 5-7, and LNA as active ingredients, together with appropriate amounts of pharmaceutical acceptable excipients and/or carriers.

**12.** A Bifidobacteria strain as defined in any of the claims 5-6 or a Lactic acid bacteria strain as defined in claim 7, for use as a probiotic.

**13.** A Bifidobacteria strain as defined in any of the claims 5-6 or a Lactic acid bacteria strain as defined in claim 7, for use as anti-obesity agent.

**14.** A Bifidobacteria strain as defined in any of the claims 5-6 or a Lactic acid bacteria strain as defined in claim 7, for use as immunomodulator agent.

**15.** A food product as defined in claim 8 or a composition as defined in any of the claims 9-11 for use as anti-obesity and immunomodulator agent.

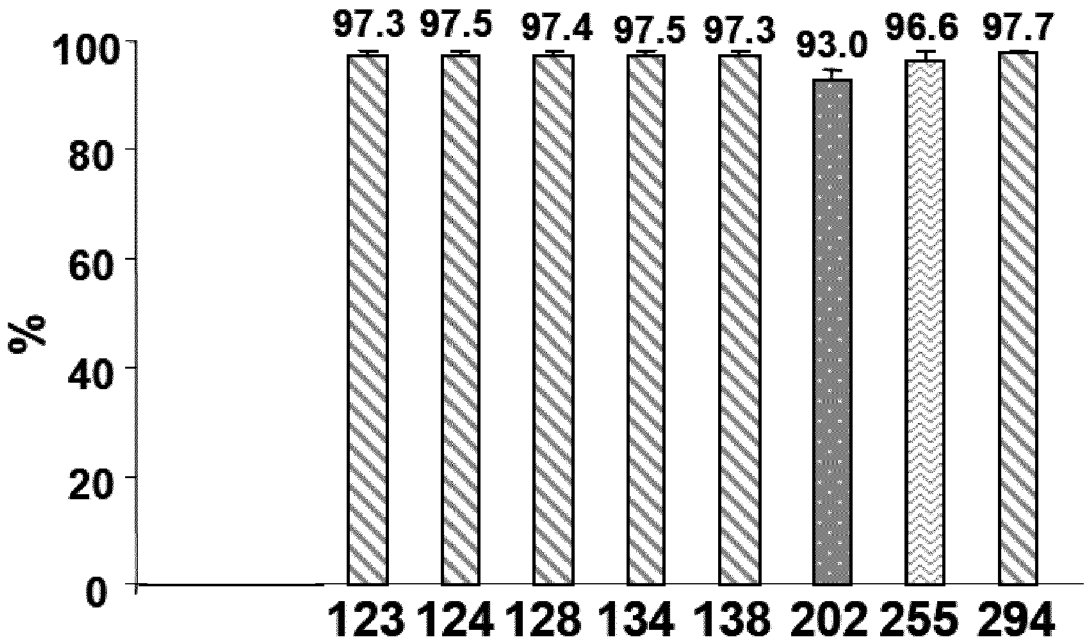
97.3
97.5
97.4
97.5
97.3
93.0
96.6
97.7
100
80
60
%
40
20
0
123
124
128
134
138
202
255
294

FIG. 1

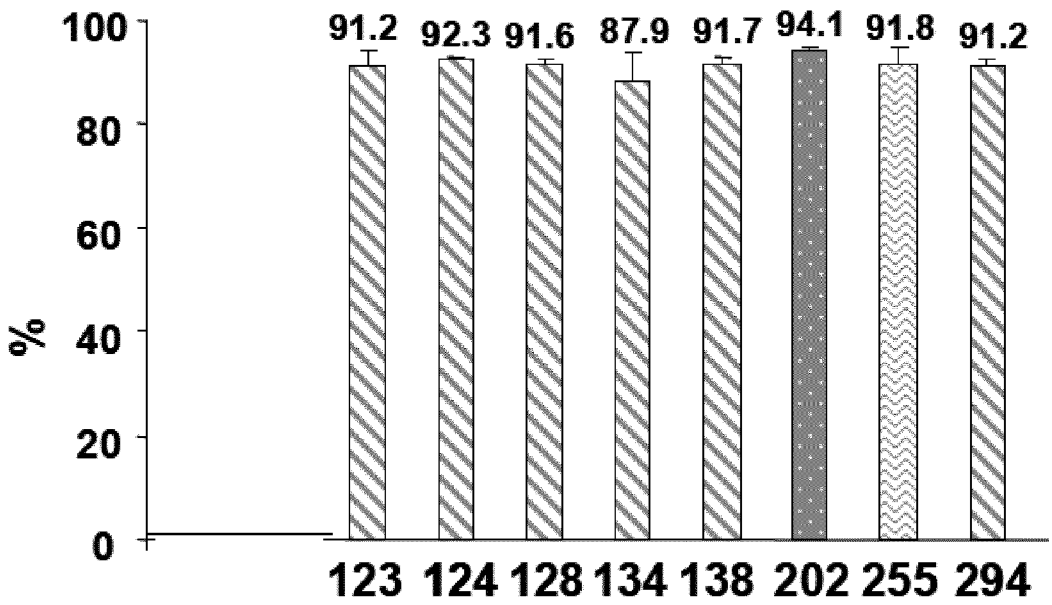
91.2
92.3
91.6
87.9
91.7
94.1
91.8
91.2
100
80
60
%
40
20
0
123
124
128
134
138
202
255
294

FIG. 2

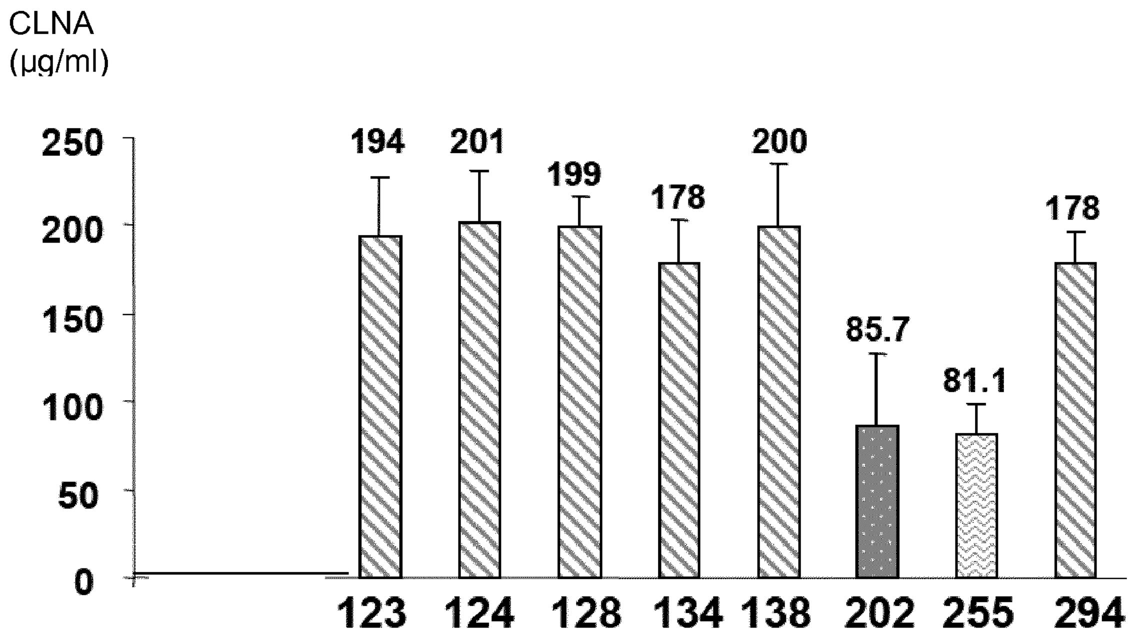
CLNA
(µg/ml)
250
200
150
100
50
0
194
201
199
178
200
85.7
81.1
178
123
124
128
134
138
202
255
294

FIG. 3

Europäisches Patentamt
European Patent Office
Office européen des brevets

# EUROPEAN SEARCH REPORT

Application Number
EP 12 19 9104

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X<br><br><br><br><br><br><br><br><br><br><br>A | JUNG, Y.-K.: "Production of conjugated linoleic acid and conjugated linolenic acid by Bifidobacterium breve JKL03 and its application", MASTER THESIS, MCGILL UNIVERSITY, CANADA , February 2005 (2005-02), pages 1-88, XP008164239, Retrieved from the Internet: URL:http://search.proquest.com/docview/305370047?accountid=29404<br>* Chapter 2 *<br>* page 29 - page 54 *<br>* see especially: *<br>* abstract *<br>* page 31, line 21 - page 32, line 5 *<br>* page 33, line 11 - line 18 *<br>* page 37, line 1 - line 5 *<br>* page 38, line 1 - page 39, line 19 *<br>* page 42, line 8 - line 17 *<br>* page 44; table 2.2 *<br>* page 46 - page 49; figures 2.2-2.5 *<br>* page 51; figure 2.7 *<br>* Chapter 3 *<br>* page 55 - page 74 *<br>* page 58, line 11 - line 15 *<br>* page 60, line 3 - line 5 *<br>* page 62, line 16 - page 63, line 9 *<br>* page 64, line 12 - page 65, line 16 *<br>* page 68; figure 3.3 *<br>* page 69; figure 3.4 *<br>* page 71; figure 3.6 *<br>* page 73; table 3.2 *<br>* page 74, line 7 - line 13 *<br>-----<br>-/-- | 1-3<br><br><br><br><br><br><br><br><br><br><br>4 | INV.<br>C12P7/64<br>C12R1/01<br>C12R1/225<br>A23L1/30<br><br>TECHNICAL FIELDS SEARCHED (IPC)<br>C12P<br>A23L<br>A61K |

~~The present search report has been drawn up for all claims~~

| Place of search | Date of completion of the search | Examiner |
|---|---|---|
| Berlin | 19 August 2013 | Fuchs, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 19 9104

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GAO, Y.-T. ET AL.: "Using alpha-linolenic acid to produce conjugated linolenic acid by fermenting Lactobacillus bulgaricus", DALIAN-GONGYE-DAXUE-XUEBAO = JOURNAL OF DALIAN POLYTECHNIC UNIVERSITY, vol. 31, no. 4, 15 July 2012 (2012-07-15), pages 235-238, XP008164237, | 1-3 | |
| A | * abstract * | 4 | |
| A | DATABASE WPI Week 201242 Thomson Scientific, London, GB; AN 2011-A54753 XP002711389, -& KR 2010 0135612 A (UNIV KOREA RESEARCH & BUSINESS FOUND) 27 December 2010 (2010-12-27) * abstract * | 1-4 | |

TECHNICAL FIELDS SEARCHED (IPC)

~~The present search report has been drawn up for all claims~~

| Place of search | Date of completion of the search | Examiner |
|---|---|---|
| Berlin | 19 August 2013 | Fuchs, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 12 19 9104

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-4

☐ The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 12 19 9104

The Search Division considers that the present European patent application does not comply with the requirements of unity of invention and relates to several inventions or groups of inventions, namely:

1. claims: 1-4

a process for preparing an isomer of conjugated linolenic acid (CLNA) from linolenic acid (LNA) in a medium comprising at least the fat portion from animal or vegetal origin contained in a milk-based product, said fat portion containing linolenic acid, the method comprising adding to the medium at least one strain used in dairy or agroalimentary industry, selected from the group consisting of a lactic acid bacteria (LAB) and a Bifidobacteria, said strain characterized by:
i) being able to grow to an optical density of at least 0.9 OD600, determined at 48 hours after initial of growth in anaerobiosis, in a Man Rogosa Sharpe broth medium, as well as in a medium comprising at least the fat portion, from animal or vegetal origin, contained in a milk-based product, both media comprising at least 0.3 mg/ml of linolenic acid in the medium and
ii) being able to produce CLNA in both media

---

2. claims: 5, 8-15(all partially)

a Bifidobacteria strain, which is a strain of Bifidobacterium breve selected from the group consisting of:
- Bifidobacterium breve deposited in the Colección Espanola de Cultivos Tipo (CECT) under the Depositor's reference ORD0123, or a mutant or variant thereof,
- Bifidobacterium breve deposited in the CECT under the Depositor's reference ORD0124, or a mutant or variant thereof,
- Bifidobacterium breve deposited in the CECT under the Depositor's reference ORD0128, or a mutant or variant thereof,
- Bifidobacterium breve deposited in the CECT under the Depositor's reference ORD0134, or a mutant or variant thereof,
- Bifidobacterium breve deposited in the CECT under the Depositor's reference ORD0138, or a mutant or variant thereof,
- Bifidobacterium breve deposited in the CECT under the Depositor's reference ORD0294, or a mutant or variant thereof,
a food product comprising an effective amount of at least one of the strains as defined in any of the claim 5, and LNA as active ingredients, together with appropriate amounts of other edible ingredients or carriers,
a nutritional composition comprising a nutritionally effective amount of at least one of the strains as defined in any of the claim 5, and LNA as active ingredients, together with appropriate amounts of other appropriate

Europäisches Patentamt
European Patent Office
Office européen des brevets

# LACK OF UNITY OF INVENTION
# SHEET B

Application Number
EP 12 19 9104

The Search Division considers that the present European patent application does not comply with the requirements of unity of invention and relates to several inventions or groups of inventions, namely:

edible ingredients or carriers,
a pharmaceutical composition comprising a therapeutically effective amount of at least one of the strains as defined in any of the claim 5, and LNA as active ingredients, together with appropriate amounts of pharmaceutical acceptable excipients and/or carriers,
a Bifidobacteria strain as defined in any of the claim 5, for use as a probiotic,
a Bifidobacteria strain as defined in any of the claim 5, for use as anti-obesity agent,
a Bifidobacteria strain as defined in any of the claim 5, for use as immunomodulator agent,
a food product as defined in claim 8 or a composition as defined in any of the claims 9-11 for use as anti-obesity and immunomodulator agent

---

3. claims: 6(completely); 5, 8-15(partially)

a Bifidobacteria strain, which is a strain of Bifidobacterium bifidum deposited in the CECT under the under the Depositor's reference ORD0202, or a mutant or variant thereof,
a food product comprising an effective amount of at least one of the strains as defined in any of the claim 6 and LNA as active ingredients, together with appropriate amounts of other edible ingredients or carriers,
a nutritional composition comprising a nutritionally effective amount of at least one of the strains as defined in any of the claim 6, and LNA as active ingredients, together with appropriate amounts of other appropriate edible ingredients or carriers,
a pharmaceutical composition comprising a therapeutically effective amount of at least one of the strains as defined in any of the claim 6, and LNA as active ingredients, together with appropriate amounts of pharmaceutical acceptable excipients and/or carriers,
a Bifidobacteria strain as defined in any of the claim 6, for use as a probiotic,
a Bifidobacteria strain as defined in any of the claim 6, for use as anti-obesity agent,
a Bifidobacteria strain as defined in any of the claim 6, for use as immunomodulator agent,
a food product as defined in claim 8 or a composition as defined in any of the claims 9-11 for use as anti-obesity and immunomodulator agent

---

4. claims: 7(completely); 8-15(partially)

a LAB strain, which is a strain of Lactobacillus oris

Europäisches Patentamt
European Patent Office
Office européen des brevets

# LACK OF UNITY OF INVENTION SHEET B

Application Number
EP 12 19 9104

The Search Division considers that the present European patent application does not comply with the requirements of unity of invention and relates to several inventions or groups of inventions, namely:

deposited in the CECT under the under the Depositor's reference ORD0255, or a mutant or variant thereof,
a food product comprising an effective amount of at least one of the strains as defined in any of the claim 7 and LNA as active ingredients, together with appropriate amounts of other edible ingredients or carriers,
a nutritional composition comprising a nutritionally effective amount of at least one of the strains as defined in any of the claim 7, and LNA as active ingredients, together with appropriate amounts of other appropriate edible ingredients or carriers,
a pharmaceutical composition comprising a therapeutically effective amount of at least one of the strains as defined in any of the claim 7, and LNA as active ingredients, together with appropriate amounts of pharmaceutical acceptable excipients and/or carriers,
a Lactic acid bacteria strain as defined in claim 7, for use as a probiotic,
a LAB strain as defined in claim 7, for use as anti-obesity agent,
a LAB strain as defined in claim 7, for use as immunomodulator agent,
a food product as defined in claim 8 or a composition as defined in any of the claims 9-11 for use as anti-obesity and immunomodulator agent

---

## ANNEX TO THE EUROPEAN SEARCH REPORT ON EUROPEAN PATENT APPLICATION NO. EP 12 19 9104

This annex lists the patent family members relating to the patent documents cited in the above-mentioned European search report. The members are as contained in the European Patent Office EDP file on The European Patent Office is in no way liable for these particulars which are merely given for the purpose of information.

19-08-2013

| Patent document cited in search report | Publication date | Patent family member(s) | Publication date |
|---|---|---|---|
| KR 20100135612 A | 27-12-2010 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **GORISSEN et al.** Production of conjugated linoleic and linolenic acid isomers by Bifidobacterium species. *Appl. Microbiol Biotechnol,* 2010, vol. 87, 2257-2266
- **HENNESSY et al.** The Production of Conjugated α-Linolenic, γ-Linolenic and Stearidonic Acids by Strains of Bifidobacteria and Propionibacteria. *Lipids,* 2012, vol. 47, 313-327
- **GORISSEN et al.** Microbial production of conjugated linoleic and linolenic acids in fermented foods: Technological bottlenecks. *Eur. J. Lipid Sci. Technol.,* 2012, vol. 114, 486-491
- **MIRANDA et al.** cis-9, trans-11, cis-15 and cis-9, trans-13, cis-15 CLNA mixtures activates PPARα in HEK293 and reduces triacylglycerols in 3T3-L1 cells. *Lipids,* 2011, vol. 46 (11), 1006-1012
- **JIMÉNEZ E et al.** Complete genome sequence of Bifidobacterium breve CECT 7263, a strain isolated from human milk. *J Bacteriol.,* 2012, vol. 194 (14), 3762-3
- **Y. NEBRA et al.** A new selective medium for Bifidobacterium spp. *Applied and Enviromental Microbiology,* 1999, vol. 65, 5173-6
- **HENNESSY et al.** The Production of Conjugated α-Linolenic, γ-Linolenic and Stearidonic Acids by Strains of Bifidobacteria and Propionibacteria. *Lipids,* 10 December 2011
- **GORISSEN et al.** Microbial production of conjugated linoleic and linolenic acids in fermented foods: Technological bottlenecks. *Eur. J. Lipid Sci. Technol.,* 2012
- **MIRANDA J et al.** cis-9,trans-11 ,cis-15 and cis-9,trans-13,cis-15 CLNA mixture activates PPARα in HEK293 and reduces triacylglycerols in 3T3-L1 cells. *Lipids,* 09 October 2011, vol. 46 (11), 1005-12
- **MIRANDA J et al.** A comparison between CLNA and CLA effects on body fat, serum parameters and liver composition. *J Physiol Biochem.,* March 2009, vol. 65 (1), 25-32
- **MARTORELL P et al.** Caenorhabditis elegans as a Model To Study the Effectiveness and Metabolic Targets of Dietary Supplements Used for Obesity Treatment: The Specific Case of a Conjugated Linoleic Acid Mixture (Tonalin. *J Agric Food Chem,* 07 November 2012, vol. 60 (44), 11071-9

SEQUENCE LISTING

<110> LABORATORIOS ORDESA, S.L.

<120> A microbiological method for obtaining conjugated linolenic acid from linolenic acid, and bacterial strains for performing the method

<130> P2453EP00

<160> 12

<170> PatentIn version 3.5

<210> 1
<211> 254
<212> DNA
<213> Bifidobacterium breve

<400> 1
```
ctttgcggca tgggatgggg tcgcgtccta tcagcttgat ggcggggtaa cggcccacca      60

tggcttcgac gggtagccgg cctgagaggg cgaccggcca cattgggact gagatacggc     120

ccagactcct acgggaggca gcagtgggga atattgcaca atgggcgcaa gcctgatgca     180

gcgacgccgc gtgagggatg gaggccttcg ggttgtaaac ctcttttgtt agggagcaag     240

gcatttttg ttga                                                        254
```

<210> 2
<211> 652
<212> DNA
<213> Bifidobacterium breve

<400> 2
```
aatagctcct ggaaacgggt ggtaatgccg gatgctccat cacaccgcat ggtgtgttgg      60

gaaagccttt gcggcatggg atggggtcgc gtcctatcag cttgatggcg gggtaacggc     120

ccaccatggc ttcgacgggt agccggcctg agagggcgac cggccacatt gggactgaga     180

tacggcccag actcctacgg gaggcagcag tggggaatat tgcacaatgg gcgcaagcct     240

gatgcagcga cgccgcgtga gggatggagg ccttcgggtt gtaaacctct tttgttaggg     300

agcaaggcat tttttgttga gtgtaccttt cgaataagca ccggctaact acgtgccagc     360

agccgcggta atacgtaggg tgcaagcgtt atccggaatt attgggcgta aagggctcgt     420

aggcggttcg tcgcgtccgg tgtgaaagtc catcgcttaa cggtggatcc gcgccgggta     480

cgggcgggct tgagtgcggt aggggagact ggaattcccg gtgtaacggt ggaatgtgta     540

gatatcggga agaacaccaa tggcgaaggc aggtctctgg gccgttactg acgctgagga     600

gcgaaagcgt ggggagcgaa caggattaga taccctggta gtccacgccg ta             652
```

<210> 3
<211> 761
<212> DNA
<213> Bifidobacterium breve

<400> 3
gatggcgggg taacggccca ccatggcttc gacgggtagc cggcctgaga gggcgaccgg 60

ccacattggg actgagatac ggcccagact cctacgggag gcagcagtgg ggaatattgc 120

acaatgggcg caagcctgat gcagcgacgc cgcgtgaggg atggaggcct tcgggttgta 180

aacctctttt gttagggagc aaggcatttt ttgttgagtg tacctttcga ataagcaccg 240

gctaactacg tgccagcagc cgcggtaata cgtagggtgc aagcgttatc cggaattatt 300

gggcgtaaag ggctcgtagg cggttcgtcg cgtccggtgt gaaagtccat cgcttaacgg 360

tggatccgcg ccgggtacgg gcgggcttga gtgcggtagg ggagactgga attcccggtg 420

taacggtgga atgtgtagat atcgggaaga acaccaatgg cgaaggcagg tctctgggcc 480

gttactgacg ctgaggagcg aaagcgtggg gagcgaacag gattagatac cctggtagtc 540

cacgccgtaa acggtggatg ctggatgtgg ggcccgttcc acgggttccg tgtcggagct 600

aacgcgttaa gcatcccgcc tggggagtac ggccgcaagg ctaaaactca aagaaattga 660

cgggggcccg cacaagcggc ggagcatgcg gattaattcg atgcaacgcg aagaacctta 720

cctgggcttg acatgttccc gacgatccca gagatggggt t 761

<210> 4
<211> 716
<212> DNA
<213> Bifidobacterium breve

<400> 4
gcggcatggg atggggtcgc gtcctatcag cttgatggcg ggtaacggc ccaccatggc 60

ttcgacgggt agccggcctg agagggcgac cggccacatt gggactgaga tacggcccag 120

actcctacgg gaggcagcag tggggaatat tgcacaatgg gcgcaagcct gatgcagcga 180

cgccgcgtga gggatggagg ccttcgggtt gtaaacctct tttgttaggg agcaaggcat 240

tttttgttga gtgtaccttt cgaataagca ccggctaact acgtgccagc agccgcggta 300

atacgtaggg tgcaagcgtt atccggaatt attgggcgta aagggctcgt aggcggttcg 360

tcgcgtccgg tgtgaaagtc catcgcttaa cggtggatcc gcgccgggta cgggcgggct 420

tgagtgcggt aggggagact ggaattcccg gtgtaacggt ggaatgtgta gatatcggga 480

agaacaccaa tggcgaaggc aggtctctgg gccgttactg acgctgagga gcgaaagcgt 540

ggggagcgaa caggattaga taccctggta gtccacgccg taaacggtgg atgctggatg 600

tggggcccgt tccacgggtt ccgtgtcgga gctaacgcgt taagcatccc gcctggggag 660

tacggccgca aggctaaaac tcaaagaaat tgacgggggc ccgcacaagc ggcgga 716

<210> 5
<211> 496
<212> DNA
<213> Bifidobacterium breve

<400> 5

ttgcggcatg ggatggggtc gcgtcctatc agcttgatgg cggggtaacg gcccaccatg 60

gcttcgacgg gtagccggcc tgagagggcg accggccaca ttgggactga gatacggccc 120

agactcctac gggaggcagc agtggggaat attgcacaat gggcgcaagc ctgatgcagc 180

gacgccgcgt gagggatgga ggccttcggg ttgtaaacct cttttgttag ggagcaaggc 240

attttttgtt gagtgtacct ttcgaataag caccggctaa ctacgtgcca gcagccgcgg 300

taatacgtag ggtgcaagcg ttatccggaa ttattgggcg taaagggctc gtaggcggtt 360

cgtcgcgtcc ggtgtgaaag tccatcgctt aacggtggat ccgcgccggg tacgggcggg 420

cttgagtgcg gtaggggaga ctggaattcc cggtgtaacg gtggaatgtg tagatatcgg 480

gaagaacacc aatggc 496

<210> 6
<211> 424
<212> DNA
<213> Bifidobacterium bifidum

<400> 6
tcctggaaac gggtggtaat gccggatgtt ccacatgatc gcatgtgatt gtgggaaaga 60

ttctatcggc gtgggatggg gtcgcgtcct atcagcttgt tggtgaggta acggctcacc 120

aaggcttcga cgggtagccg gcctgagagg gcgaccggcc acattgggac tgagatacgg 180

cccagactcc tacgggaggc agcagtgggg aatattgcac aatgggcgca agcctgatgc 240

agcgacgccg cgtgagggat ggaggccttc gggttgtaaa cctcttttgt ttgggagcaa 300

gccttcgggt gagtgtacct ttcgaataag cgccggctaa ctacgtgcca gcagccgcgg 360

taatacgtag ggcgcaagcg ttatccggat ttattgggcg taaagggctc gtaggcggct 420

cgtc 424

<210> 7
<211> 777
<212> DNA
<213> Lactobacillus oris

<400> 7
ggggataaca tttggaaaca ggtgctaata ccgcataact tggaaaacca catggttttc 60

caataaaaga tggtttcggc tatcactttg ggatgggccc gcggtgcatt agctagttgg 120

taaggtaacg gcttaccaag gcgatgatgc atagccgagt tgagagactg atcggccaca 180

atggaactga gacacggtcc atactcctac gggaggcagc agtagggaat cttccacaat 240

gggcgcaagc ctgatggagc aacaccgcgt gagtgaagaa gggtttcggc tcgtaaaact 300

ctgttgttgg agaagaacgt gcgtaagagt aactgtttac gcagtgacgg tatccaacca 360

gaaagtcacg gctaactacg tgccagcagc cgcggtaata cgtaggtggc aagcgttatc 420

cggatttatt gggcgtaaag cgagcgcagg cggttgctta ggtctgatgt gaaagccttc 480

ggcttaaccg aagaagtgca tcggaaaccg ggcgacttga gtgcagaaga ggacagtgga 540

actccatgtg tagcggtgga atgcgtagat atatggaaga acaccagtgg cgaaggcggc 600

tgtctggtct gcaactgacg ctgaggctcg aaagcatggg tagcgaacag gattagatac 660

cctggtagtc catgccgtaa acgatgagtg ctaggtgttg gagggtttcc gcccttcagt 720

gccgaagcta acgcattaag cactccgcct ggggagtacg accgcaaggt tgaaact 777

<210> 8
<211> 493
<212> DNA
<213> Bifidobacterium breve

<400> 8
gcggcatggg atggggtcgc gtcctatcag cttgatggcg gggtaacggc ccaccatggc 60

ttcgacgggt agccggcctg agagggcgac cggccacatt gggactgaga tacggcccag 120

actcctacgg gaggcagcag tggggaatat tgcacaatgg gcgcaagcct gatgcagcga 180

cgccgcgtga gggatggagg ccttcgggtt gtaaacctct tttgttaggg agcaaggcat 240

tttttgttga gtgtaccttt cgaataagca ccggctaact acgtgccagc agccgcggta 300

atacgtaggg tgcaagcgtt atccggaatt attgggcgta aagggctcgt aggcggttcg 360

tcgcgtccgg tgtgaaagtc catcgcttaa cggtggatcc gcgccgggta cgggcgggct 420

tgagtgcggt aggggagact ggaattcccg gtgtaacggt ggaatgtgta gatatcggga 480

agaacaccaa tgg 493

<210> 9
<211> 24
<212> DNA
<213> Artificial

<220>
<223> Forward primer

<400> 9
tggctcagga cgaacgctgg cggc 24

<210> 10
<211> 24
<212> DNA
<213> Artificial

<220>
<223> Reverse primer

<400> 10
cctactgctg cctcccgtag gagt 24

<210> 11
<211> 20
<212> DNA
<213> Artificial

<220>

<223> Forward primer

<400> 11
aatacatgca agtcgaacga 20

<210> 12
<211> 20
<212> DNA
<213> Artificial

<220>
<223> Reverse primer

<400> 12
ttaacccaac atctcacgac 20